(19) Europäisches Patentamt
European Patent Office
Office européen des brevets

(11) EP 4 671 232 A1

(12) EUROPEAN PATENT APPLICATION

(43) Date of publication:
**31.12.2025 Bulletin 2026/01**

(21) Application number: **24184512.2**

(22) Date of filing: **26.06.2024**

(51) International Patent Classification (IPC):
***C07C 209/16*** (2006.01) ***C07C 209/62*** (2006.01)
***C07C 211/13*** (2006.01) ***C07C 303/28*** (2006.01)

(52) Cooperative Patent Classification (CPC):
(C-Sets available)
**C07C 209/16; C07C 209/62; C07C 211/13;**
**C07C 303/28;** C07B 2200/13 (Cont.)

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(71) Applicant: **Bayer Aktiengesellschaft**
**51373 Leverkusen (DE)**

(72) Inventor: **The designation of the inventor has not yet been filed**

(74) Representative: **BIP Patents**
**c/o Bayer Intellectual Property GmbH**
**Alfred-Nobel-Straße 50**
**40789 Monheim am Rhein (DE)**

(54) **A SCALABLE PROCESS FOR THE MANUFACTURE OF 2,2-BIS(AMINOMETHYL) PROPANE-1,3-DIAMINE TETRAHYDROCHLORIDE**

(57) The present disclosure relates to a process for the preparation of 2,2-bis(aminomethyl)propane-1,3-diamine tetrahydrochloride, to intermediates involved in said process such as pentaerythritol tetratosylate and tetrabenzyl-neopentyltetraamine, to a crystalline salt of the intermediate tetrabenzyl-neopentyltetraamine tetrahydrochloride as well as to a crystalline tetraacetate salt of of 2,2-bis(aminomethyl)propane-1,3-diamine.

EP 4 671 232 A1

(52) Cooperative Patent Classification (CPC): (Cont.)

C-Sets
**C07C 209/16, C07C 211/27;**
**C07C 209/62, C07C 211/13;**
**C07C 303/28, C07C 309/73**

## Description

## FIELD

The present disclosure relates to a process for the preparation of 2,2-bis(aminomethyl)propane-1,3-diamine tetrahydrochloride, to intermediates involved in said process such as pentaerythritol tetratosylate and tetrabenzyl-neopentyltetraamine, to a crystalline salt of the intermediate tetrabenzyl-neopentyltetraamine tetrahydrochloride as well as to a crystalline tetraacetate salt of of 2,2-bis(aminomethyl)propane-1,3-diamine as well as their use in the preparation of Gadoquatrane, i.e. 2-[4,10-bis(carboxylatomethyl)-7-[1-oxo-1-[[2-oxo-2-[[3-[[2-[2-[4, 7,10-tris(carboxylatomethyl)-1, 4, 7,10-tetrazacyclododec-1-yl]propanoylamino]acetyl]amino]-2,2-bis[[[2-[2-[4,7,10-tris(carboxylatomethyl)-1,4,7,10-tetrazacyclododec-1-yl]propanoylamino]acetyl]amino]methyl]propyl]amino]ethyl]amino]propan-2-yl]-1,4,7,10-tetrazacyclododec-1-yl]acetate, gadolinium(3+) and 2,2-bis(aminomethyl)propane-1,3-diamine tetrahydrochloride.

## BACKGROUND

### 1. Introduction

One of the most important topics in the field of Radiology is the development of new contrast agents. Due to the toxicity of gadolinium, there is an increased need for the provision of new contrast agents which can be administered in lower doses while at the same time maintaining beneficial physician and pharmacokinetic properties such as a high relaxivity. One such new, gadolinium-based contrast agent is Gadoquatrane (IUPAC-name 2-[4,10-bis(carboxylatomethyl)-7-[1-oxo-1-[[2-oxo-2-[[3-[[2-[2-[4,7,10-tris(carboxylatomethyl)-1,4,7,10-tetrazacyclododec-1-yl]propanoylamino]acetyl]amino]-2,2-bis[[[2-[2-[4,7,10-tris(carboxylatomethyl)-1,4,7,1 0-tetrazacyclododec-1-yl]propanoylamino]acetyl]amino]methyl]propyl]amino]ethyl]amino]propan-2-yl]-1,4,7,10-tetrazacyclododec-1-yl]acetate, gadolinium(3+)), which is suitable for intra-venous administration in a single dose and in lower doses than common Gd-based contrast agents currently on the market. It is self-evident that regulatory and toxicological aspects call for a high purity of the drug substance and thus ultimately also for a high purity of the compound's key building blocks, gadolinium 10-[4-carboxy-1-methyl-2-oxo-3-azabutyl]-1,4,7,10-tetraazacyclododecan-1,4,7-triacetic acid and 2,2-bis(aminomethyl)propane-1,3-diamine tetrahydrochloride. The synthesis of Gadoquatrane has been described, for example, in WO2016/193190 and WO2024/088910.

Due to the increased demand for new and safer contrast agents which can be administered in low doses, it is expected that the demand for Gadoquatrane will lie on the multi-metric ton range. Thus, the provision of a reliable, safe and scalable process for the manufacture of 2,2-bis(aminomethyl)propane-1,3-diamine and in particular of its tetrahydrochloride salt, is critical.

### 2. Description of the prior art, problem to be solved and its solution

As outlined in Scheme 1, the synthesis of 2,2-bis(aminomethyl)propane-1,3-diamine has been described from pentaerythritol by different routes:

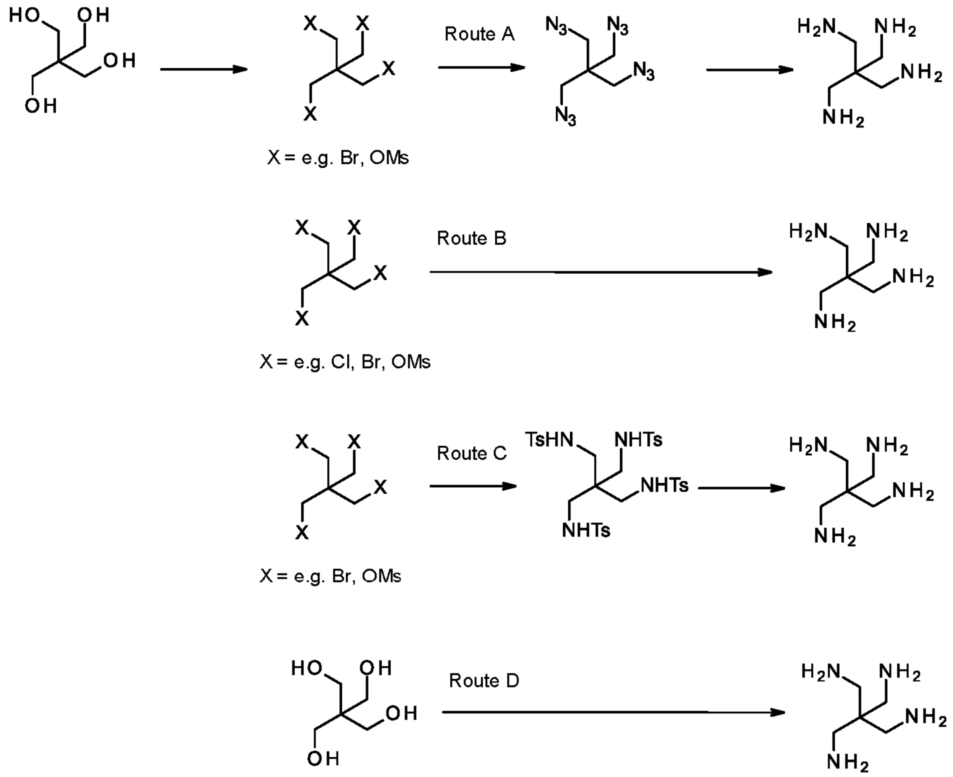


Scheme 1. Synthetic routes to 2,2-bis(aminomethyl)propane-1,3-diamine as described in the literature

Route A: the conversion of the alcohol functionalities of pentaerythritol into suitable leaving groups (i.e. bromide, mesylate) followed by reaction with an azide source at elevated temperatures yielding an intermediate tetraazide and subsequent reduction to the amine functionality either by Pd-catalyzed hydrogenation (e.g. Tetrahedron, 2003, vol. 59, # 40, p. 7983 - 7996; Chemical Communications, 2010, vol. 46, # 1, p. 142 - 144; RSC Advances, 2014, vol. 4, #43, p. 22561 - 22566; Chemical Communications, 2020, vol. 56, # 73, p. 10662 - 10665) or by treatment with lithium aluminum hydride (Journal of Organic Chemistry, 1971, vol. 36, p. 3042 - 3044).

Route B: the conversion of the alcohol functionalities of pentaerythritol into suitable leaving groups (i.e. bromide, mesylate) followed by reaction with ammonia either in ethanol (Recueil des Travaux Chimiques des Pays-Bas, 1938, vol. 57, p. 265,276) at elevated temperatures or the use of ammonium hydroxide in acetonitrile (US10155064, 2018, B2). Anderson and colleagues described the conversion of neopentylterachloride with supercritical ammonia under high pressure, partially using methanol as the co-solvent (J. Ind. Eng Chem. 2000, 39, 4011).

Route C: the conversion of the alcohol functionalities of pentaerythritol into suitable leaving groups (i.e. bromide, mesylate) followed by reaction with para-toluenesulfonamide followed by hydrolysis under very acidic and harsh conditions (180 - 200 °C, sulphuric acid, Canadian Journal of Chemistry, 1989, vol. 67, p. 1650- 1656; Journal of the Chemical Society, 1938, p. 1588-1593).

Route D: The direct conversion of pentaerythritol to the target compound by direct aminolysis in the presence of a catalyst and hydrogen at 210 °C under high pressure with 1,4-dioxane as a solvent (CN111018716, 2020, A).

While still allowing for the preparation of 2,2-bis(aminomethyl)propane-1,3-diamine, the routes described above encompass several hurdles and significant drawbacks that restrict their use in the manufacture of the desired compound in the required quantities and, more importantly, with the required purity.

Synthetic route A has been described to proceed in a batch-wise process which includes the isolation of solid 1,3-diazido-2,2-bis(azidomethyl)propane by intermediate concentration to dryness upon extractive work-up and purification by column chromatography. This complicates its amenability to scale both from a cost as well as from a safety perspective given the environmental and safety hazard posed by such a small molecule comprising four azide moieties.

The process according to synthetic route C requires harsh conditions both from a temperature point of view (180-200°C) as well as from the chemical reagents employed (strong acids), thus calling for the use of very specialized

equipment, which limits its large-scale applicability and increases the production costs.

While synthetic routes B and D may appear promising by taking advantage of cheap and abundantly available ammonia as the nitrogen source and by not requiring any additional functional group manipulation, it is unclear how and in which quality the final compound is isolated.

Further, all processes according to routes A-D have only been described to be viable on a very limited scale (in most cases, less than 10 g, in one case, up to ~50 g). Thus, the previously reported processes fail to provide a process for the preparation of 2,2-bis(aminomethyl)propane-1,3-diamine which is amenable for scale-up and capable of providing a product which may meet the high-quality standards required by pharmaceutical agencies and regulatory authorities.

Thus, there is an unmet need to provide a process for the preparation of 2,2-bis(aminomethyl)propane-1,3-diamine which:

- is reliable and amenable for scale-up in large quantities,
- avoids the use of hazardous reagents and/or the isolation of highly energetic intermediates
- is cost efficient and provides a high overall yield,
- preferably encompasses a purification process in order to provide the compound in high quality and purity to meet regulatory guidelines,
- is suitable for monitoring with analytical methods that provide for quality control and that easily allow for the detection and identification of impurities in view of the highly polar nature of the target compound which lacks any chromophores, thus severily limiting the analytical options.

The present disclosure provides a process for the preparation of 2,2-bis(aminomethyl)propane-1,3-diamine which employs benzylic amines, and in particular benzylamine as a readily available and cost-efficient amine equivalent. The target product, i.e., 2,2-bis(aminomethyl)propane-1,3-diamine can be conveniently prepared by removing the corresponding benzylic groups by hydrogenative deprotection. In particular, the process according to the present disclosure shows a high selectivity in light of potential side reactions and allows for the introduction of purification step(s) in order to provide the target compound in high quality and purity.

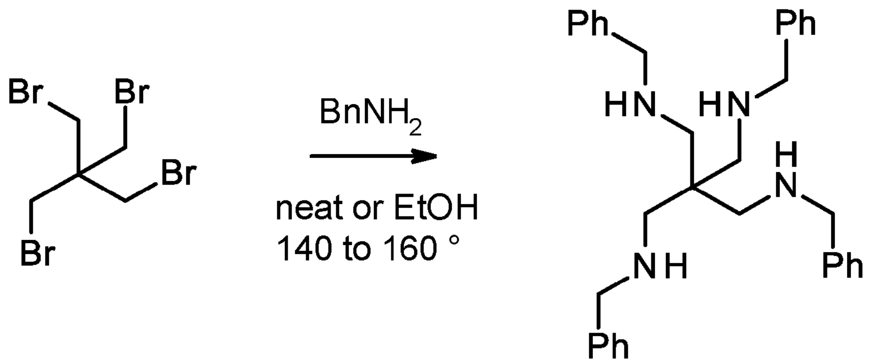


Scheme 2: Reaction of 1,3-dibromo-2,2-bis(bromomethyl)-propane with benzylamine as described in the literature (Recueil des Travaux Chimiques des Pays-Bas, 1938, vol. 57, p. 265-276 and Tur. J. Chem. 2001, 25, 293).

The conversion of the tetrabromide 1,3-dibromo-2,2-bis(bromomethyl)-propane to the corresponding tetrabenzyl amine in ethanol as solvent at 160 °C has been described in the following literature: Recueil des Travaux Chimiques des Pays-Bas, 1938, vol. 57, p. 265-276. In addition, Cakir and colleagues (Tur. J. Chem. 2001, 25, 293) described the transformation in neat benzylamine at temperatures between 140 and 160 °C albeit on low scale and using cancerogenic benzene in the process. While the latter route described analytical properties such as $^1$H NMR and IR, as with the above described routes A-D to 2,2-bis(aminomethyl)propane-1,3-diamine, no yield has been reported and the routes are not suitable for scale-up (Scheme 2)

Instead of 1,3-dibromo-2,2-bis(bromomethyl)-propane, the present disclosure uses pentaerythritol tetratosylate as a starting material for the preparation of 2,2-bis(aminomethyl)propane-1,3-diamine. The use of 1,3-dibromo-2,2-bis(bromomethyl)-propane is inconvenient because it must be prepared either from pentaerythritol tetratosylate - thus adding at least one more step to the synthetic sequence - and/or its synthesis involves the use of hazardous and environmentally questionable reagents such as phosphorus tribromide ($PBr_3$). Further, unlike 1,3-dibromo-2,2-bis(bromomethyl)-propane, pentaerythritol tetratosylate is readily and conveniently available in large quantities at a reasonable cost - its synthesis has been previously described in the literature: a) Journal of the American Chemical Society, 2012, vol. 134, # 32, p. 13145 - 13147; b) CN104262271, 2016, B.

Surprisingly, the present disclosure provides a process that allows for the preparation of 2,2-bis(aminomethyl) propane-1,3-diamine and that overcomes the above-described disadvantages and drawbacks of the previously reported processes.

In particular, it has surprisingly been found that a clean conversion of pentaerythritol tetratosylate to the tetrabenzylamine is achievable at lower (as low as 140°C) reaction temperatures than those employed in the processes previously reported in the literature. Further, it has surprisingly been found that the process can be carried out in a large scale safely and that it allows for the efficient removal of polar side components and inorganic salts. Finally, the process according to the present disclosure allows for the isolation of the tetrahydrochloride salt of tetrabenzyl-neopentyltetraamine via a well-defined crystallization process in high yields and purity.

It has surprisingly been found that the removal of the benzylic groups of tetrabenzyl-neopentyltetraamine to yield 2,2-bis(aminomethyl)propane-1,3-diamine can be readily achieved by a hydrogenation process which allows for a smooth separation and removal of deleterious inorganic salts and the convenient isolation of the desired product as the tetraacetate salt and as a hydrochloride salt in high yield and exceptional purity by well-defined crystallization processes.

**DEFINITIONS**

The term "substituted" means that one or more hydrogen atoms on the designated atom or group are replaced with a selection from the indicated group, provided that the designated atom's normal valency under the existing circumstances is not exceeded. Combinations of substituents and/or variables are permissible.

The term "optionally substituted" means that the number of substituents can be equal to or different from zero.

The term "comprising" when used in the specification includes "consisting of" and "consisting essentially of".

If within the present text any item is referred to as "as mentioned herein", it means that it may be mentioned anywhere in the present text.

The terms as mentioned in the present text have the following meanings:
The term "halogen atom" means a fluorine, chlorine, bromine or iodine atom, particularly a fluorine, chlorine or bromine atom.

The compounds of the present disclosure may contain one or more asymmetric centers, depending upon the location and nature of the various substituents desired. Asymmetric carbon atoms may be present in the (R) or (S) configuration, which can result in racemic mixtures, mixtures in which one enantiomer is present in a greater amount than the other enantiomer, or single enantiomers in the case of a single asymmetric center. In the case of multiple stereogenic centers, diastereomeric mixtures, single diastereomers or single enantiomers can be synthesized. In certain instances, asymmetry may also be present due to restricted rotation about a given bond, axial chirality or coordination of the metal center.

The optical isomers can be obtained by resolution of the racemic mixtures according to conventional processes, for example, by the formation of diastereoisomeric salts using an optically active acid or base or formation of covalent diastereomers. Examples of appropriate acids are tartaric, diacetyltartaric, ditoluoyltartaric and camphorsulfonic acid. Mixtures of diastereoisomers can be separated into their individual diastereomers on the basis of their physical and/or chemical differences by methods known in the art, for example, by chromatography or fractional crystallisation. The optically active bases or acids are then liberated from the separated diastereomeric salts. A different process for separation of optical isomers involves the use of chiral chromatography (e.g., chiral HPLC columns), with or without conventional derivatisation, optimally chosen to maximise the separation of the enantiomers. Suitable chiral HPLC columns are manufactured by Daicel, e.g., Chiracel OD and Chiracel OJ among many others, all routinely selectable. Enzymatic separations, with or without derivatisation, are also useful. The optically active compounds of this disclosure can likewise be obtained by chiral syntheses utilizing optically active starting materials and/or reagents and catalysts.

In order to describe different types of isomers reference is made to IUPAC Rules Section E (Pure Appl Chem 45, 11-30, 1976).

The present disclosure includes all possible stereoisomers of the compounds of the present disclosure as single stereoisomers, or as any mixture of said stereoisomers, e.g. R- or S-isomers, or diastereoisomers, in any ratio. Isolation of a single stereoisomer, e.g. a single enantiomer or a single diastereomer, of a compound of the present disclosure may be achieved by any suitable state of the art method as described herein, such as chromatography, especially chiral chromatography, for example.

Further, the compounds of the present disclosure can exist as N-oxides, which are defined in that at least one nitrogen of the compounds of the present disclosure is oxidized. The present disclosure includes all such possible N-oxides.

The present disclosure also relates to useful forms of the compounds as disclosed herein, such as hydrates,

solvates, salts, in particular pharmaceutically acceptable salts, and co-precipitates.

The compounds of the present disclosure can exist as a hydrate, or as a solvate, wherein the compounds of the present disclosure contain polar solvents, in particular water, methanol or ethanol for example as structural element of the crystal lattice of the compounds. The amount of polar solvents, in particular water, may exist in a stoichiometric or non-stoichiometric ratio. In the case of stoichiometric solvates, *e.g.* a hydrate, hemi-, (semi-), mono-, sesqui-, di-, tri-, tetra-, penta- *etc.* solvates or hydrates, respectively, are possible. The present disclosure includes all such hydrates or solvates.

Further, the compounds of the present disclosure can exist in the form of a salt. Said salt may be either an inorganic or organic addition salt, particularly any pharmaceutically acceptable inorganic or organic addition salt, customarily used in pharmaceutical formulations.

The present disclosure includes all possible salts of the compounds of the present disclosure as single salts, or as any mixture of said salts, in any ratio.

In the present text, in particular in the Experimental Section, for the synthesis of intermediates and of examples of the present disclosure, when a compound is mentioned as a salt form with the corresponding base or acid, the exact stoichiometric composition of said salt form, as obtained by the respective preparation and/or purification process, is, unless stated otherwise, unknown.

This applies analogously to cases in which synthesis intermediates or example compounds or salts thereof have been obtained, by the preparation and/or purification processes described, as solvates, such as hydrates with (if defined) unknown stoichiometric composition, unless stated otherwise.

In the context of the present disclosure, the terms "N,N'-dibenzyl-2,2-bis[(benzylamino)methyl]propane-1,3-diamine" and "tetrabenzyl neopentyltetraamine" may be used interchangeably and refer to the same compound, i.e.:

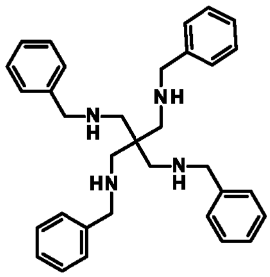

In the context of the present disclosure, the terms "neopentyltetraamine" and "2,2-bis(aminomethyl)propane-1,3-diamine" may be used interchangeably and refer to the same compound, i.e.:

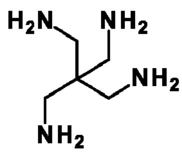

## DESCRIPTION

In accordance with a first aspect, the present disclosure relates to a process for the preparation of 2,2-bis(aminomethyl)propane-1,3-diamine comprising

(i) providing sulfonic ester-activated pentaerythritol,
(ii) reacting the sulfonic ester-activated pentaerythritol with a benzylic amine to yield a benzylic neopentyltetraamine,
(iii) removing the benzylic group from the benzylic neopentyltetraamine and
(iv) isolating 2,2-bis(aminomethyl)propane-1,3-diamine as a hydrochloride salt, preferably as a tetrahydrochloride salt.

## FURTHER EMBODIMENTS

In step (i), sulfonic ester-activated pentaerythritol is provided. Any sulfonic acid suitable for the activation of the alcohol group in pentaerythritol can be used as long as it is suitable for the reaction with a benzylic amine to yield a benzylic neopentyltetraamine. The sulfonic acid can be an alkyl or an aryl sulfonic acid. Preferably, the sulfonic acid is an aryl sulfonic acid. Also preferably, the sulfonic acid is selected from the group consisting of methanesulfonic acid, p-toluenesulfonic acid, p-nitrosulfonic acid and benzenesulfonic acid. Also preferably, the sulfonic acid is selected from

the group consisting of p-toluenesulfonic acid, p-nitrosulfonic acid and benzenesulfonic acid. More preferably, the sulfonic acid is p-toluenesulfonic acid (Tosyl, Ts). Thus, in a further embodiment of the first aspect, providing sulfonic ester-activated pentaerythritol in step (i) comprises the provision of pentaerythritol-tetratosylate.

The provision of sulfonic ester-activated pentaerythritol in step (i) can be achieved by (i-a) providing pentaerythritol and (i-b) reacting pentaerythritol with a sulfonyl halide to sulfonic ester-activated pentaerythritol.

Thus, in a further embodiment of the first aspect, the present disclosure relates to a process for the preparation of 2,2-bis(aminomethyl)propane-1,3-diamine comprising

(i) providing sulfonic ester-activated pentaerythritol by

(i-a) providing pentaerythritol and
(i-b) reacting pentaerythritol with a sulfonyl halide to sulfonic ester-activated pentaerythritol,

(ii) reacting the sulfonic ester-activated pentaerythritol with a benzylic amine to yield a benzylic neopentyltetraamine,
(iii) removing the benzylic group from the benzylic neopentyltetraamine and
(iv) isolating 2,2-bis(aminomethyl)propane-1,3-diamine as a hydrochloride salt, preferably as a tetrahydrochloride salt.

Any sulfonyl halide can be used as long as it allows for the preparation of a sulfonic ester-activated pentaerythritol which is suitable for the reaction with a benzylic amine to yield a benzylic neopentyltetraamine. The sulfonyl halide can be an alkyl or aryl sulfonyl halide. Preferably, the sulfonyl halide is an aryl sulfonyl halide. Preferably, the sulfonyl halide is an aryl sulfonyl chloride. Preferably, the sulfonyl halide is selected from the group consisting of methanesulfonyl chloride, p-toluenesulfonyl chloride, p-nitrosulfonyl chloride and benzenesulfonyl chloride. More preferably, the sulfonyl halide is p-toluenesulfonyl chloride.

In a further embodiment of the first aspect, the present disclosure relates to a process for the preparation of 2,2-bis(aminomethyl)propane-1,3-diamine comprising

(i) providing pentaerythritol tetratosylate,
(ii) reacting pentaerythritol tetratosylate with a benzylic amine to yield a benzylic neopentyltetraamine,
(iii) removing the benzylic group from the benzylic neopentyltetraamine and
(iv) isolating 2,2-bis(aminomethyl)propane-1,3-diamine as a hydrochloride salt, preferably as a tetrahydrochloride salt.

In a further embodiment of the first aspect, the present disclosure relates to a process for the preparation of 2,2-bis(aminomethyl)propane-1,3-diamine comprising

(i) providing pentaerythritol tetratosylate by

(i-a) providing pentaerythritol and
(i-b) reacting pentaerythritol with p-toluenesulfonyl chloride to pentaerythritol tetratosylate,

(ii) reacting pentaerythritol tetratosylate with a benzylic amine to yield a benzylic neopentyltetraamine,
(iii) removing the benzylic group from the benzylic neopentyltetraamine and
(iv) isolating 2,2-bis(aminomethyl)propane-1,3-diamine as a hydrochloride salt, preferably as a tetrahydrochloride salt.

In step (ii), the sulfonic ester-activated pentaerythritol is reacted with a benzylic amine to yield a benzylic neopentyltetraamine. Pentaerythritol can be activated with an alkyl or an aryl sulfonic acid. Preferably, pentaerythritol is activated as a sulfonic ester such as methanesulfonic ester, more preferably as an aryl sulfonic acid which is prepared using a sulfonic acid selected from the group consisting of p-toluenesulfonic acid, p-nitrosulfonic acid and benzenesulfonic acid. Preferably, pentaerythritol is activated with p-toluenesulfonic acid. More preferably, the sulfonic ester-activated pentaerythritol is pentaerythritol tetratosylate.

In reacting the sulfonic ester-activated pentaerythritol with a benzylic amine to yield a benzylic neopentyltetraamine, there is no limitation to the nature of the benzylic amine as long as it is capable of providing a benzylic neopentyltetraamine. Preferably, the benzylic amine is selected from the group consisting of benzyl amine, 4-methoxybenzylamine and 3,4-dimethoxybenzylamine. More preferably, the benzylic amine is benzyl amine.

The reaction is preferably carried out at temperatures ranging of from 100 °C to 180 °C, more preferably of from 120 °C to 160 °C and most preferably of from 130 °C to 150 °C. The reaction is carried out with a reaction time ranging of

from 1 h to 48 h, more prerably of from 4 h to 24 h, most preferably of from 6 h to 10 h.

While the use of solvents and co-solvents is not excluded as long as they allow for the preparation of a benzylic neopentyltetraamine, the reaction is preferably carried out in neat benzylic amine. Thus, when benzylamine is used, the reaction is preferably carried out in neat benzylamine. Also preferably, the reaction is carried out using 4 equivalents to 50 equivalents of the benzylic amine, preferably 10 equivalents to 40 equivalents of the benzylic amine, more preferably 15 equivalents to 25 equivalents of the benzylic amine. More preferably, the reaction is carried out using 4 equivalents to 50 equivalents of benzylamine, preferably 10 equivalents to 40 equivalents of benzylamine, more preferably 15 equivalents to 25 equivalents of benzylamine.

Optionally, the reaction mixture comprising a benzylic neopentyltetraamine may be extracted to remove any excess of the benzylic amine by adding water and an organic solvent and adjusting the pH value. Preferably, the organic solvent is an aromatic solvent or an ether or a mixture of two or more thereof. Preferably, the organc solvent is an aromatic solvent selected from o-xylene, m-xylene, p-xylene, toluene and ethylbenzene or an ether selected from diethylether, diisopropylether, tetrahydrofurane, methyltetrahydrofurane and methyl-tert-butyl ether or a mixture of two or more thereof. More preferably, the organic solvent is methyl-tert-butyl ether or toluene.

Preferably, the pH value is set between 6 and 9, more preferably between 7 and 8. Optionally, the benzylic neopentyltetraamine may be further purified by solvent swapping followed by precipitation of a salt of the benzylic neopentyltetraamine by addition of a mineral acid. Preferably, the solvent used is an alcohol. More preferably, the solvent used is methanol or ethanol. Preferably, the salt of the benzylic neopentyltetraamine is precipitated using hydrochloric acid. Optionally, the salt of the benzylic neopentyltetraamine can be converted to a solution of the free base in an organic solvent by partitioning between the organic solvent a basic aqueous solution. Preferably, the organic solvent is methyl tert-butyl ether. Preferably, the basic aqueous solution is an aqueous sodium hydroxide solution.

In step (iii), the benzylic group from the benzylic neopentyltetraamine produced in step (ii) is removed. Any suitable method can be employed as long as it is suitable for the removal of the benzylic group. Preferred suitable reagents for the removal of the benzylic group are metal-based hydrogenation catalysts. Preferably, metal-based hydrogenation catalysts are catalysts based on a transition metal selected from Pd, Pt, Ir, Ru and Rh. Preferably, metal-based hydrogenation catalysts are based on Pd or Pt, such as palladium on carbon (Pd/C), palladium hydroxide on carbon ($Pd(OH)_2/C$) and $Pt_2O$. Preferably, the reduction is carried out using hydrogen gas in the presence of a suitable metal-based hydrogenation catalyst. More preferably, the catalyst is palladium on carbon, i.e., Pd/C or palladium hydroxide on carbon ($Pd(OH)_2/C$). Optionally, step (iii) may comprise isolating 2,2-bis(aminomethyl)propane-1,3-diamine as an acetate salt, preferably as a tetraacetate salt. Preferably, step (iii) comprises isolating 2,2-bis(aminomethyl)propane-1,3-diamine as an acetate salt, preferably as a tetraacetate salt. Preferably, the tetraacetate salt of 2,2-bis(aminomethyl)propane-1,3-diamine is isolated in crystalline form.

If step (iii) comprises the preferred isolation of 2,2-bis(aminomethyl)propane-1,3-diamine as an acetate salt, preferably as a tetraacetate salt, the hydrogenation, i.e. the removal of the benzylic group, is preferably carried out in the presence of acetic acid, preferably using from 3 to 6 equivalents of acetic acid, more preferably of from 4 to 5 equivalents of acetic acid.

If step (iii) comprises the preferred isolation of 2,2-bis(aminomethyl)propane-1,3-diamine as an acetate salt, preferably as a tetraacetate salt, the hydrogenation is preferably carried out in a mixture consisting of two solvents, the first solvent preferably being an alcohol, and the second solvent preferably being water. Preferably, the first solvent is a C1-C5 alcohol, i.e., a linear or branched, saturated, monovalent hydrocarbon group having 1, 2, 3, 4 or 5 carbon atoms in which one or more of the hydrogen atoms are replaced, identically or differently, with a hydroxy (OH) group. Preferably, the first solvent is an alcohol selected from methanol, ethanol and 2-propanol (isopropanol). More preferably, the first solvent is 2-propanol (isopropanol). Preferably, the ratio of the first solvent and of the second solvent is in the range of from 1:0 to 0:1, more preferably of from 20:1 to 1:1, more preferably of from 15:1 to 3:1, more preferably of from 12:1 to 6:1, more preferably of from 10:1 to 8:1.

The preferred isolation of 2,2-bis(aminomethyl)propane-1,3-diamine as a tetraacetate salt in step (iii) may involve the partial removal of the reaction solvent or solvents. Preferably, said partial removal of the reaction solvent or solvents is carried out by distillation and may include addition of solvents to achieve the final conditions of the crystallization (vide infra). Preferably, the temperature during destillative work-up lies below 65 °C, more preferably below 55 °C, more preferably below 45 °C.

If step (iii) comprises the preferred isolation of 2,2-bis(aminomethyl)propane-1,3-diamine as an acetate salt, preferably as a tetraacetate salt in crystalline form, the water content of the reaction mixture for crystallization preferably ranges of from 10 to 0 % by weight, more preferably of from 5 to 0 % by weight, more preferably of from 3 to 0 % by weight.

The preferred isolation of 2,2-bis(aminomethyl)propane-1,3-diamine as a tetraacetate salt in crystalline form may comprise the addition of additional amounts of acetic acid. Preferably, additional amount of acetic acid ranges of from 8 to 0.5 equivalents more preferably of from 3 to 1 equivalents. In order to induce crystallization, the reaction mixture may optionally be cooled down. Preferably, a gradual cooling ramp is employed. Preferably, the cooling ramp comprises cooling the reaction mixture from 65 °C to -20 °C over the course of 2 hours to 6 hours, more preferably from 40 °C to -5 °C over the

course 3 hours to 5 hours. The cooling ramp may optionally include holding times during the cooling of the crystallization mass. Preferably, the cooling process comprises holding the crystallization mass at a temperature in the range of from 15 to 25 °C for a time in the range of from 1 to 3 hours, preferably followed by an additional holding time at a temperature in the range of from -5 to 5 °C for a time in the range of from 1 to 3 hours. Preferably, the cooling process comprises holding the crystallization mass at about 20 °C for about 2 hours, preferably followed by an additional holding time at about 0 °C for about 2 hours.

Step (iv) comprises the isolation of 2,2-bis(aminomethyl)propane-1,3-diamine as a hydrochloride salt, preferably as a tetrahydrochloride salt. Preferably, the tetraacetate salt of 2,2-bis(aminomethyl)propane-1,3-diamine is isolated in step (iii) and then, in step (iv), converted to its tetrahydrochloride salt and isolated.

In step (iv), 2,2-bis(aminomethyl)propane-1,3-diamine is isolated as a hydrochloride salt, preferably as a tetrahydrochloride salt. For the isolation of 2,2-bis(aminomethyl)propane-1,3-diamine as a tetrahydrochloride salt, 2,2-bis(aminomethyl)propane-1,3-diamine is provided, preferably as its tetraacetate salt after the removal of the benzylic group from the benzylic neopentyltetraamine in step (iii).

If 2,2-bis(aminomethyl)propane-1,3-diamine is provided as its tetraacetate salt prior to its conversion to the hydrochloride salt of 2,2-bis(aminomethyl)propane-1,3-diamine, the tetraacetate salt of 2,2-bis(aminomethyl)propane-1,3-diamine is provided in a suitable solvent or solvent mixture. Preferably said suitable solvent is water.

For the isolation of 2,2-bis(aminomethyl)propane-1,3-diamine as a tetrahydrochloride salt, 2,2-bis(aminomethyl)propane-1,3-diamine or the tetraacetate salt of 2,2-bis(aminomethyl)propane-1,3-diamine is reacted with hydrochloric acid. Preferably, the isolation of 2,2-bis(aminomethyl)propane-1,3-diamine as a tetrahydrochloride salt comprises the reaction of 2,2-bis(aminomethyl)propane-1,3-diamine or of the tetraacetate salt of 2,2-bis(aminomethyl)propane-1,3-diamine in a suitable solvent or solvent mixture as described above with an excess of hydrochloric acid ranging of from 3 equivalents to 20 equivalents, preferably with an excess of hydrochloric acid ranging of from 4 equivalents to 10 equivalents, more preferably with an excess of hydrochloric acid ranging of from 4.5 equivalents to 7 equivalents, more preferably with about 5 equivalents of hydrochloric acid.

The isolation of 2,2-bis(aminomethyl)propane-1,3-diamine as a tetrahydrochloride salt in step (iv) may comprise a purification step. Said purification step may comprise suspending and/or dissolving the crude tetrahydrochloride salt of 2,2-bis(aminomethyl)propane-1,3-diamine in a first solvent or solvent mixture and then adding a second solvent or solvent mixture to induce crystallization. Preferably, said first solvent is water. Preferably, the second solvent is an alcohol selected from methanol, ethanol, n-propanol, 2-propanol (isopropanol) and n-butanol. More preferably, the second solvent is isopropanol. Preferably, the ratio of the first solvent and of the second solvent ranges of from 1:10 to 0:1, more preferably of from 1:5 to 1:1, more preferably of from 1:4 to 1:1.25, more preferably of from 1:3 to 1:2. If the first solvent is water and the second solvent is isopropanol, the ratio of water and of isopropanol ranges of from 1:10 to 0:1, more preferably of from 1:5 to 1:1, more preferably of from 1:4 to 1:1.25, more preferably of from 1:3 to 1:2. Temperature adjustments may be beneficial to induce crystallization, i.e., the crude tetrahydrochloride salt of 2,2-bis(aminomethyl)propane-1,3-diamine may be suspended and/or dissolved in the first solvent or solvent mixture and heated prior to adding the second solvent or solvent mixture. Preferably, the second solvent is added at a temperature in the range of from 40 °C to 100 °C, more preferably in the range of from 40 °C to 60 °C, more preferably in the range of from 45 °C to 55 °C. Preferably, the first solvent is water and the second solvent which is isopropanol added at a temperature in the range of from 40 °C to 100 °C, more preferably in the range of from 40 °C to 60 °C, more preferably in the range of from 45 °C to 55 °C. Particularly preferred is a temperature of about 50 °C.

Subsequently, the mixture may be cooled down to induce crystallization. If cooled down to induce crystallization, in step (iii) the mixture may be optionally and/or preferably cooled down to a temperature in the range of from - 10 °C to 70 °C, more preferably in the range of from 0 °C to 50 °C, more preferably in the range of from 10 °C to 40 °C, more preferably in the range of from 15 °C to 25 °C. Particularly preferred is a temperature of about 20°C.

Thus, in accordance with a further embodiment of the first aspect, the present disclosure relates to a process for the preparation of 2,2-bis(aminomethyl)propane-1,3-diamine comprising

(i) providing sulfonic ester-activated pentaerythritol,
(ii) reacting the sulfonic ester-activated pentaerythritol with a benzylic amine to yield a benzylic neopentyltetraamine,
(iii) removing the benzylic group from the benzylic neopentyltetraamine and optionally isolating 2,2-bis(aminomethyl)propane-1,3-diamine as an acetate salt, preferably as a tetraacetate salt,
(iv) converting the tetraacetate salt of 2,2-bis(aminomethyl)propane-1,3-diamine to its tetrahydrochloride salt and isolating 2,2-bis(aminomethyl)propane-1,3-diamine as a hydrochloride salt, preferably as a tetrahydrochloride salt.

Thus, in accordance with a further embodiment of the first aspect, the present disclosure relates to a process for the preparation of 2,2-bis(aminomethyl)propane-1,3-diamine comprising

(i) providing sulfonic ester-activated pentaerythritol,

(ii) reacting the sulfonic ester-activated pentaerythritol with a benzylic amine to yield a benzylic neopentyltetraamine,
(iii) removing the benzylic group from the benzylic neopentyltetraamine and optionally isolating 2,2-bis(aminomethyl) propane-1,3-diamine as an acetate salt, preferably as a tetraacetate salt,
(iv-a) converting the tetraacetate salt of 2,2-bis(aminomethyl)propane-1,3-diamine to its tetrahydrochloride salt and
(iv-b) isolating 2,2-bis(aminomethyl)propane-1,3-diamine as a hydrochloride salt, preferably as a tetrahydrochloride salt.

In step (iv), 2,2-bis(aminomethyl)propane-1,3-diamine is isolated as a hydrochloride salt, preferably as a tetrahydrochloride salt. Preferably, 2,2-bis(aminomethyl)propane-1,3-diamine obtained in step (iii) is provided as an acetate salt, preferably as a tetraacetate salt, preferably in crystalline form, i.e., as a crystalline tetraacetate salt.
In order to convert the tetraacetate salt to the tetrahydrochloride salt, the acetate salt is dissolved in water and subsequently reacted with reacted with hydrochloric acid. Preferably, the isolation of 2,2-bis(aminomethyl)propane-1,3-diamine as a tetrahydrochloride salt comprises the reaction of 2,2-bis(aminomethyl)propane-1,3-diamine tetra acetate with hydrochloric acid, ranging from 3 equivalents to 20 equivalents, more preferably of ranging from 4 equivalents to 10 equivalents, even more preferably of from 4.5 equivalents to 7 equivalents.
The addition of concentrated aqueous hydrochloric acid is carried out preferably ranging from 0 °C to 50 °C, more preferably ranging of from 10 °C to 40 °C, most preferably of from 20 °C to 30 °C.
The addition of concentrated aqueous hydrochloric acid is preferably carried out in a time frame ranging from 10 min to 300 min, more preferably of ranging from 30 min to 240 min, most preferably of ranging from 60 min to 120 min.
Preferably the tetrahydrochloride salt is isolated by subsequent addition of an organic solvent, more preferably an alcohol, even more preferably an alcohol selected from methanol, ethanol, n-propanol, 2-propanol (isopropanol) and n-butanol. Most preferably, the second solvent is isopropanol.
Preferably the ratio of water and isopropanol (v/v) ranges of from 1:10 to 0:1, more preferably of from 1:5 to 1:1, even more preferably of from 1:4 to 1:2.
Temperature adjustments may be beneficial for purification, and the solvent mixture may be heated and subsequently cooled prior to isolation. Preferably, the mixture is heated ranging from 20 °C to 80 °C, more preferably ranging of from 40 °C to 60 °C. The mixture is heated preferably of from 10 min to 300 min, more preferably of from 30 min to 120 min. Washing of the isolated solid is preferably carried out with a mixture of water and isopropanol. Preferably the ratio of water and isopropanol (v/v) ranges of from 1:10 to 0:1, more preferably of from 1:6 to 1:1, even more preferably of from 1:5 to 1:2.

## Crystalline acetate salt of 2,2-bis(aminomethyl)propane-1,3-diamine

In a further aspect, the present disclosure relates to the tetraacetate salt of 2,2-bis(aminomethyl)propane-1,3-diamine. In particular, the present disclosure relates to the tetraacetate salt of 2,2-bis(aminomethyl)propane-1,3-diamine in crystalline form, i.e., to a crystalline form of the tetraacetate salt of 2,2-bis(aminomethyl)propane-1,3-diamine.
The crystalline tetraacetate salt of 2,2-bis(aminomethyl)propane-1,3-diamine may be characterized by analytical methods well known in the field of the pharmaceutical industry for characterizing solids. Such methods comprise but are not limited to powder X-ray diffraction (PXRD/XRPD), Fourier transform Infrared (FTIR) spectroscopy, differential scanning calorimetry (DSC), thermogravimetric analysis (TGA) and dynamic vapour sorption (DVS). The crystalline tetraacetate salt of 2,2-bis(aminomethyl)propane-1,3-diamine may be characterized by one of the aforementioned methods or by combining two or more of them. In particular, crystalline tetraacetate salt of 2,2-bis(aminomethyl) propane-1,3-diamine may be characterized by one of the following embodiments or by combining two or more of the following embodiments.
The crystalline tetraacetate salt of 2,2-bis(aminomethyl)propane-1,3-diamine is characterized by having a powder X-ray diffractogram comprising reflections at 2-Theta angles of (9.8 ± 0.2)°, (11.4 ± 0.2)° and (20.6 ± 0.2)°, when measured at room temperature with Cu-Kalpha$_1$ radiation having a wavelength of 0.15419 nm. Preferably, the crystalline tetraacetate salt of 2,2-bis(aminomethyl)propane-1,3-diamine is characterized by having a powder X-ray diffractogram comprising reflections at 2-Theta angles of (9.8 ± 0.2)°, (11.4 ± 0.2)°, (20.6 ± 0.2)°, (22.4 ± 0.2)° and (25.0 ± 0.2)°, when measured at room temperature with Cu-Kalpha$_1$ radiation having a wavelength of 0.15419 nm. Preferably, the crystalline tetraacetate salt of 2,2-bis(aminomethyl)propane-1,3-diamine is characterized by having a powder X-ray diffractogram comprising reflections at 2-Theta angles of (9.8 ± 0.2)°, (11.4 ± 0.2)°, (17.9 ± 0.2)°, (18.8 ± 0.2)°, (20.6 ± 0.2)°, (22.4 ± 0.2)° and (25.0 ± 0.2)°, when measured at room temperature with Cu-Kalpha$_1$ radiation having a wavelength of 0.15419 nm. Preferably, the crystalline tetraacetate salt of 2,2-bis(aminomethyl)propane-1,3-diamine is characterized by having a powder X-ray diffractogram comprising reflections at 2-Theta angles of (9.8 ± 0.2)°, (11.4 ± 0.2)°, (17.9 ± 0.2)°, (18.8 ± 0.2)°, (20.6 ± 0.2)°, (22.4 ± 0.2)°, (24.4 ± 0.2)°, (25.0 ± 0.2)°, (27.7 ± 0.2)° and (32.1 ± 0.2)°, when measured at room temperature with Cu-Kalpha$_1$ radiation having a wavelength of 0.15419 nm.
Alternatively or additionally, the crystalline tetraacetate salt of 2,2-bis(aminomethyl)propane-1,3-diamine is

characterized by having a powder X-ray diffractogram essentially the same as displayed in Figure 5 of the present disclosure, when measured at room temperature with Cu-Kalpha$_1$ radiation having a wavelength of 0.15419 nm.

Alternatively or additionally, the crystalline tetraacetate salt of 2,2-bis(aminomethyl)propane-1,3-diamine is characterized by having a Fourier transform Infrared spectrum comprising bands at wavenumbers of (655 $\pm$ 2) cm$^{-1}$, (982 $\pm$ 2) cm$^{-1}$, and (1403 $\pm$ 2) cm$^{-1}$, when measured at room temperature with a diamond ATR cell. Preferably, the crystalline tetraacetate salt of 2,2-bis(aminomethyl)propane-1,3-diamine is characterized by having a Fourier transform Infrared spectrum comprising peaks at wavenumbers of (558 $\pm$ 2) cm$^{-1}$, (655 $\pm$ 2) cm$^{-1}$, (923 $\pm$ 2) cm$^{-1}$, (982 $\pm$ 2) cm$^{-1}$ and (1403 $\pm$ 2) cm$^{-1}$, when measured at room temperature with a diamond ATR cell. Preferably, the crystalline tetraacetate salt of 2,2-bis(aminomethyl)propane-1,3-diamine is characterized by having a Fourier transform Infrared spectrum comprising bands at wavenumbers of (558 $\pm$ 2) cm$^{-1}$, (655 $\pm$ 2) cm$^{-1}$, (923 $\pm$ 2) cm$^{-1}$, (982 $\pm$ 2) cm$^{-1}$, (1016 $\pm$ 2) cm$^{-1}$, (1403 $\pm$ 2) cm$^{-1}$ and (1516 $\pm$ 2) cm$^{-1}$, when measured at room temperature with a diamond ATR cell. Preferably, the crystalline tetraacetate salt of 2,2-bis(aminomethyl)propane-1,3-diamine is characterized by having a Fourier transform Infrared spectrum comprising bands at wavenumbers of (558 $\pm$ 2) cm$^{-1}$, (618 $\pm$ 2) cm$^{-1}$, (655 $\pm$ 2) cm$^{-1}$, (848 $\pm$ 2) cm$^{-1}$, (923 $\pm$ 2) cm$^{-1}$, (982 $\pm$ 2) cm$^{-1}$, (1016 $\pm$ 2) cm$^{-1}$, (1332 $\pm$ 2) cm$^{-1}$, (1403 $\pm$ 2) cm-$^{1}$ and (1516 $\pm$ 2) cm$^{-1}$, when measured at room temperature with a diamond ATR cell.

Alternatively, the crystalline tetraacetate salt of 2,2-bis(aminomethyl)propane-1,3-diamine is characterized by having a Fourier transform Infrared spectrum essentially the same as displayed in Figure 6 of the present disclosure, when measured at room temperature with a diamond ATR cell.

Alternatively or additionally, the crystalline tetraacetate salt of 2,2-bis(aminomethyl)propane-1,3-diamine is characterized by having a Raman spectrum comprising peaks at wavenumbers of (926 $\pm$ 2) cm$^{-1}$, (1412 $\pm$ 2) cm$^{-1}$ and (2929 $\pm$ 2) cm$^{-1}$, when measured at room temperature with a laser at a wavelength of 1064nm. Preferably, the crystalline tetraacetate salt of 2,2-bis(aminomethyl)propane-1,3-diamine is characterized by having a Raman spectrum comprising bands at wavenumbers of (661 $\pm$ 2) cm$^{-1}$, (926 $\pm$ 2) cm$^{-1}$, (1412 $\pm$ 2) cm$^{-1}$, (1491 $\pm$ 2) cm$^{-1}$ and (2929 $\pm$ 2) cm$^{-1}$, when measured at room temperature with a laser at a wavelength of 1064nm. Preferably, the crystalline tetraacetate salt of 2,2-bis(aminomethyl)propane-1,3-diamine is characterized by having a Raman spectrum comprising peaks at wavenumbers of (661 $\pm$ 2) cm$^{-1}$, (926 $\pm$ 2) cm$^{-1}$, (1047 $\pm$ 2) cm$^{-1}$, (1351 $\pm$ 2) cm$^{-1}$, (1412 $\pm$ 2) cm$^{-1}$, (1491 $\pm$ 2) cm-$^{1}$ and (2929 $\pm$ 2) cm$^{-1}$, when measured at room temperature with a laser at a wavelength of 1064nm. Preferably, the crystalline tetraacetate salt of 2,2-bis(aminomethyl)propane-1,3-diamine is characterized by having a Raman spectrum comprising peaks at wavenumbers of (661 $\pm$ 2) cm$^{-1}$, (745 $\pm$ 2) cm$^{-1}$, (926 $\pm$ 2) cm$^{-1}$, (1034 $\pm$ 2) cm$^{-1}$, (1047 $\pm$ 2) cm$^{-1}$, (1351 $\pm$ 2) cm$^{-1}$, (1412 $\pm$ 2) cm$^{-1}$, (1491 $\pm$ 2) cm$^{-1}$, (2929 $\pm$ 2) cm$^{-1}$ and (2986 $\pm$ 2) cm$^{-1}$, when measured at room temperature with a laser at a wavelength of 1064nm.

Alternatively, the crystalline tetraacetate salt of 2,2-bis(aminomethyl)propane-1,3-diamine is characterized by having a Raman spectrum essentially the same as displayed in Figure 7 of the present disclosure, when measured at room temperature with a laser at a wavelength of 1064nm.

## Crystalline tetrahydrochloride salt of tetrabenzyl neopentyltetraamine

In a further aspect, the present disclosure relates to the tetrahydrochloride salt of tetrabenzyl neopentyltetraamine. In particular, the present disclosure relates to the tetrahydrochloride salt of tetrabenzyl neopentyltetraamine in crystalline form, i.e., to a crystalline form of the tetrahydrochloride salt of tetrabenzyl neopentyltetraamine.

The crystalline tetrahydrochloride salt of tetrabenzyl neopentyltetraamine may be characterized by analytical methods well known in the field of the pharmaceutical industry for characterizing solids. Such methods comprise but are not limited to powder X-ray diffraction (PXRD/XRPD), Fourier transform Infrared (FTIR) spectroscopy, differential scanning calorimetry (DSC), thermogravimetric analysis (TGA) and dynamic vapour sorption (DVS). The crystalline tetrahydrochloride salt of tetrabenzyl neopentyltetraamine may be characterized by one of the aforementioned methods or by combining two or more of them. In particular, the crystalline tetrahydrochloride salt of tetrabenzyl neopentyltetraamine may be characterized by one of the following embodiments or by combining two or more of the following embodiments.

The crystalline tetrahydrochloride salt of tetrabenzyl neopentyltetraamine is characterized by having a powder X-ray diffractogram comprising reflections at 2-Theta angles of (9.0 $\pm$ 0.2)°, (21.7 $\pm$ 0.2)° and (26.9 $\pm$ 0.2)°, when measured at room temperature with Cu-Kalpha$_1$ radiation having a wavelength of 0.15419 nm. Preferably, the crystalline tetrahydrochloride salt of tetrabenzyl neopentyltetraamine is characterized by having a powder X-ray diffractogram comprising reflections at 2-Theta angles of (9.0 $\pm$ 0.2)°, (14.5 $\pm$ 0.2)°, (21.7 $\pm$ 0.2)°, (22.8 $\pm$ 0.2)° and (26.9 $\pm$ 0.2)°, when measured at room temperature with Cu-Kalpha$_1$ radiation having a wavelength of 0.15419 nm. Preferably, the crystalline tetrahydrochloride salt of tetrabenzyl neopentyltetraamine is characterized by having a powder X-ray diffractogram comprising reflections at 2-Theta angles of (9.0 $\pm$ 0.2)°, (14.5 $\pm$ 0.2)°, (21.7 $\pm$ 0.2)°, (22.1 $\pm$ 0.2)°, (22.8 $\pm$ 0.2)°, (26.6 $\pm$ 0.2)° and (26.9 $\pm$ 0.2)°, when measured at room temperature with Cu-Kalpha$_1$ radiation having a wavelength of 0.15419 nm. Preferably, the crystalline tetrahydrochloride salt of tetrabenzyl neopentyltetraamine is characterized by having a powder X-ray diffractogram comprising reflections at 2-Theta angles of (9.0 $\pm$ 0.2)°, (14.5 $\pm$ 0.2)°, (15.9 $\pm$ 0.2)°, (19.2 $\pm$ 0.2)°,

(21.7 ± 0.2)°, (22.1 ± 0.2)°, (22.5 ± 0.2)°, (22.8 ± 0.2)°, (26.6 ± 0.2)° and (26.9 ± 0.2)°, when measured at room temperature with Cu-Kalpha$_1$ radiation having a wavelength of 0.15419 nm.

Alternatively or additionally, the crystalline tetrahydrochloride salt of tetrabenzyl neopentyltetraamine is characterized by having a powder X-ray diffractogram essentially the same as displayed in Figure 2 of the present disclosure, when measured at room temperature with Cu-Kalpha$_1$ radiation having a wavelength of 0.15419 nm.

Alternatively or additionally, the crystalline tetrahydrochloride salt of tetrabenzyl neopentyltetraamine is characterized by having a Fourier transform Infrared spectrum comprising bands at wavenumbers of (695 ± 2) cm$^{-1}$, (752 ± 2) cm$^{-1}$, and (1448 ± 2) cm$^{-1}$, when measured at room temperature with a diamond ATR cell. Preferably, the crystalline tetrahydrochloride salt of tetrabenzyl neopentyltetraamine is characterized by having a Fourier transform Infrared spectrum comprising peaks at wavenumbers of (504 ± 2) cm$^{-1}$, (695 ± 2) cm$^{-1}$, (752 ± 2) cm$^{-1}$, (952 ± 2) cm$^{-1}$ and (1448 ± 2) cm$^{-1}$, when measured at room temperature with a diamond ATR cell. Preferably, the crystalline tetrahydrochloride salt of tetrabenzyl neopentyltetraamine is characterized by having a Fourier transform Infrared spectrum comprising bands at wavenumbers of (504 ± 2) cm$^{-1}$, (695 ± 2) cm$^{-1}$, (752 ± 2) cm$^{-1}$, (767 ± 2) cm$^{-1}$, (952 ± 2) cm$^{-1}$, (1448 ± 2) cm$^{-1}$ and (1552 ± 2) cm$^{-1}$, when measured at room temperature with a diamond ATR cell. Preferably, the crystalline tetrahydrochloride salt of tetrabenzyl neopentyltetraamine is characterized by having a Fourier transform Infrared spectrum comprising bands at wavenumbers of (449 ± 2) cm$^{-1}$, (504 ± 2) cm$^{-1}$, (583 ± 2) cm$^{-1}$, (602 ± 2) cm$^{-1}$, (695 ± 2) cm$^{-1}$, (752 ± 2) cm$^{-1}$, (767 ± 2) cm$^{-1}$, (952 ± 2) cm$^{-1}$, (1448 ± 2) cm$^{-1}$ and (1552 ± 2) cm$^{-1}$, when measured at room temperature with a diamond ATR cell.

Alternatively, the crystalline tetrahydrochloride salt of tetrabenzyl neopentyltetraamine is characterized by having a Fourier transform Infrared spectrum essentially the same as displayed in Figure 3 of the present disclosure, when measured at room temperature with a diamond ATR cell.

Alternatively or additionally, the crystalline tetrahydrochloride salt of tetrabenzyl neopentyltetraamine is characterized by having a Raman spectrum comprising peaks at wavenumbers of (1005 ± 2) cm$^{-1}$, (1221 ± 2) cm$^{-1}$ and (3064 ± 2) cm$^{-1}$, when measured at room temperature with a laser at a wavelength of 1064nm. Preferably, the crystalline tetrahydrochloride salt of tetrabenzyl neopentyltetraamine is characterized by having a Raman spectrum comprising bands at wavenumbers of (620 ± 2) cm$^{-1}$, (1005 ± 2) cm$^{-1}$, (1221 ± 2) cm$^{-1}$, (1609 ± 2) cm$^{-1}$ and (3064 ± 2) cm$^{-1}$, when measured at room temperature with a laser at a wavelength of 1064nm. Preferably, the crystalline tetrahydrochloride salt of tetrabenzyl neopentyltetraamine is characterized by having a Raman spectrum comprising peaks at wavenumbers of (620 ± 2) cm$^{-1}$, (1005 ± 2) cm$^{-1}$, (1037 ± 2) cm$^{-1}$, (1221 ± 2) cm$^{-1}$, (1426 ± 2) cm$^{-1}$, (1609 ± 2) cm$^{-1}$ and (3064 ± 2) cm$^{-1}$, when measured at room temperature with a laser at a wavelength of 1064nm. Preferably, the crystalline tetrahydrochloride salt of tetrabenzyl neopentyltetraamine is characterized by having a Raman spectrum comprising peaks at wavenumbers of (620 ± 2) cm$^{-1}$, (1005 ± 2) cm$^{-1}$, (1037 ± 2) cm$^{-1}$, (1188 ± 2) cm$^{-1}$, (1221 ± 2) cm$^{-1}$, (1426 ± 2) cm$^{-1}$, (1588 ± 2) cm$^{-1}$, (1609 ± 2) cm$^{-1}$, (2966 ± 2) cm$^{-1}$ and (3064 ± 2) cm$^{-1}$, when measured at room temperature with a laser at a wavelength of 1064nm.

Alternatively, the crystalline tetrahydrochloride salt of tetrabenzyl neopentyltetraamine is characterized by having a Raman spectrum essentially the same as displayed in Figure 4 of the present disclosure, when measured at room temperature with a laser at a wavelength of 1064nm.

## DESCRIPTION OF THE FIGURES

Figure 1 displays a chromatogramm of 1,3-diamino-2,2-bis(aminomethyl)propane tetrahydrochloride prepared according to Example 1, step 4, example 4b).

Figure 2 displays the XRD spectrum of pentaerythritol-tetrabenzylamine tetrahydrochloride (Example 1, step 2).

Figure 3 displays the IR spectrum of pentaerythritol-tetrabenzylamine tetrahydrochloride (Example 1, step 2).

Figure 4 displays the Raman spectrum of pentaerythritol-tetrabenzylamine tetrahydrochloride (Example 1, step 2).

Figure 5 displays the XRD spectrum of neopentyltetraamine tetraacetate (Example 1, step 3).

Figure 6 displays the IR spectrum of neopentyltetraamine tetraacetate (Example 1, step 3).

Figure 7 displays the Raman spectrum of neopentyltetraamine tetraacetate (Example 1, step 3).

## EXPERIMENTAL SECTION

Chemical names were generated using the ACD/Name software from ACD/Labs. In some cases generally accepted names of commercially available reagents were used in place of ACD/Name generated names.

The following table 1 lists the abbreviations used in this paragraph and in the Examples section as far as they are not explained within the text body. Other abbreviations have their meanings customary *per se* to the skilled person.

**Table 1:** Abbreviations

| Abbreviation | Meaning |
|---|---|
| AcOH | acetic acid |
| ACN | acetonitrile |
| aq. | aqueous |
| AUC | Area under the curve |
| br | broad signal (NMR) |
| bw | Body weight |
| $C_{Gd}$ | Gadolinium concentration |
| CPMG | Carr-Purcell-Meiboom-Gill (MRI sequence) |
| d | doublet (NMR) |
| DAD | Diode Array Detector |
| DBU | 1,8-diazabicyclo(5.4.0)undec-7-ene |
| DCM | Dichloromethane |
| dd | doublet of doublet (NMR) |
| DIPEA | diisopropylethylamine |
| DMA | *N,N*-dimethylacetamide |
| DMAP | 4-dimethylaminopyridine |
| DMF | *N,N*-dimethylformamide |
| DMSO | Dimethylsulfoxide |
| ESI | electrospray (ES) ionization |
| EtOAc | ethyl acetate |
| EtOH | ethanol |
| FLASH | Fast Low Angle Shot |
| Gd | Gadolinium |
| h | hour(s) |
| HCl | hydrogen chloride, hydrochloric acid |
| HPLC | high performance liquid chromatography |
| ICP-MS | inductively coupled plasma mass spectrometry |
| IR | Inversion Recovery |
| LC-MS | liquid chromatography-mass spectrometry |
| m | multiplet (NMR) |
| mCPBA | meta-Chloroperoxybenzoic acid |
| MeCN | Acetonitrile |
| MeOH | Methanol |
| min | minute(s) |

(continued)

| Abbreviation | Meaning |
|---|---|
| MS | mass spectrometry |
| MTBE | Methyl-tert-butylether |
| MRI | Magnetic Resonance Imaging |
| MWD | multiple wavelength detector |
| NaCl | sodium chloride |
| NMR | Nuclear Magnetic Resonance spectroscopy : chemical shifts (δ) are given in ppm. The chemical shifts were corrected by setting the DMSO signal to 2.50 ppm using unless otherwise stated. |
| q | quartet (NMR) |
| $r_i$ | relaxivities (where i=1, 2) |
| $r_1$ or r1 | reflect how T1 relaxation rates change as a function of contrast agent concentration (slope T1 versus Gd concentration) |
| $r_2$ or r2 | reflect how T2 relaxation rates change as a function of contrast agent concentration (slope T2 versus Gd concentration) |
| $R_1$ | longitudinal relaxation rate ($1/r_1$ ) |
| $R_2$ | transversal relaxation rate ($1/ r_2$) |
| Rt or RT | room temperature |
| $R_t$, Rt | retention time |
| s | singulet (NMR) |
| sat. | saturated |
| SFC | supercritical fluid chromatography |
| $T_1$ or T1 | longitudinal relaxation time (T1 relaxation time) |
| $T_2$ or T2 | transversal relaxation time (T2 relaxation time) |
| t | triplet (NMR) |
| td | triplet of doublet (NMR) |
| TEA | triethylamine |
| TFA | trifluoroacetic acid |
| THF | tetrahydrofuran |
| δ | chemical shift |

Other abbreviations have their meanings customary *per se* to the skilled person.

The various aspects of the disclosure described in this application are illustrated by the following examples which are not meant to limit the disclosure in any way.

The example testing experiments described herein serve to illustrate the present disclosure and the disclosure is not limited to the examples given.

## EXPERIMENTAL SECTION - GENERAL PART

All reagents, for which the synthesis is not described in the experimental part, are either commercially available, or are known compounds or may be formed from known compounds by known methods by a person skilled in the art.

The compounds and intermediates produced according to the methods of the disclosure may require purification. Purification of organic compounds is well known to the person skilled in the art and there may be several ways of purifying the same compound. In some cases, no purification may be necessary. In some cases, the compounds may be purified by crystallization. In some cases, impurities may be removed by trituration using a suitable solvent. In some cases, the compounds may be purified by chromatography, particularly flash column chromatography, using for example prepacked silica gel cartridges, e.g. Biotage SNAP cartridges KP-Sil® or KP-NH® in combination with a Biotage autopurifier system

(SP4® or Isolera Four®) and eluents such as gradients of hexane/ethyl acetate or DCM/methanol. In flash column chromatography, unmodified ("regular") silica gel may be used as well as aminophase functionalized silica gel. If reference is made to flash column chromatography or to flash chromatography in the experimental section without specification of a stationary phase, regular silica gel was used.

In some cases, the compounds may be purified by preparative HPLC using for example a Waters autopurifier equipped with a diode array detector and/or on-line electrospray ionization mass spectrometer in combination with a suitable prepacked reverse phase column and eluents such as gradients of water and acetonitrile which may contain additives such as trifluoroacetic acid, formic acid or aqueous ammonia.

In some cases, purification methods as described above can provide those compounds of the present disclosure which possess a sufficiently basic or acidic functionality in the form of a salt, such as, in the case of a compound of the present disclosure which is sufficiently basic, a trifluoroacetate or formate salt for example, or, in the case of a compound of the present disclosure which is sufficiently acidic, an ammonium salt for example. A salt of this type can either be transformed into its free base or free acid form, respectively, by various methods known to the person skilled in the art, or be used as salts in subsequent biological assays. It is to be understood that the specific form (*e.g.* salt, free base etc.) of a compound of the present disclosure as isolated and as described herein is not necessarily the only form in which said compound can be applied to a biological assay in order to quantify the specific biological activity.

### NMR Spectra

The multiplicities of proton signals in $^1$H NMR spectra given in the following paragraphs reflect the observed signal form and do not take into account any higher-order signal phenomena. As a rule, the chemical shift data refers to the center of the signal in question. In the case of wide multiplets, a range is specified. Signals hidden by solvent or water were either assigned tentatively or are not listed. Strongly broadened signals - *e.g.* caused by rapid rotation of molecular moieties or by interchanging protons - have also been assigned tentatively (often referred to as a broad multiplet or broad singlet) or are not shown.

The $^1$H-NMR data of selected compounds are listed in the form of $^1$H-NMR peaklists. Therein, for each signal peak the $\delta$ value in ppm is given, followed by the signal intensity, reported in round brackets. The $\delta$ value-signal intensity pairs from different peaks are separated by commas. Therefore, a peaklist is described by the general form: $\delta_1$ (intensity$_1$), $\delta_2$ (intensity$_2$), ... , $\delta_i$ (intensity$_i$), ... , $\delta_n$ (intensity$_n$).

The intensity of a sharp signal correlates with the height (in cm) of the signal in a printed NMR spectrum. When compared with other signals, this data can be correlated to the real ratios of the signal intensities. In the case of broad signals, more than one peak, or the center of the signal along with their relative intensity, compared to the most intense signal displayed in the spectrum, are shown. A $^1$H-NMR peaklist is similar to a classical $^1$H-NMR readout, and thus usually contains all the peaks listed in a classical NMR interpretation. Moreover, similar to classical $^1$H-NMR printouts, peaklists can show solvent signals, signals derived from stereoisomers of the particular target compound, peaks of impurities, $^{13}$C satellite peaks, and/or spinning sidebands. The peaks of stereoisomers, and/or peaks of impurities are typically displayed with a lower intensity compared to the peaks of the target compound (*e.g.*, with a purity of >90%). Such stereoisomers and/or impurities may be typical for the particular manufacturing process, and therefore their peaks may help to identify a reproduction of the manufacturing process on the basis of "by-product fingerprints". An expert who calculates the peaks of the target compound by known methods (MestReC, ACD simulation, or by use of empirically evaluated expectation values), can isolate the peaks of the target compound as required, optionally using additional intensity filters. Such an operation would be similar to peak-picking in classical $^1$H-NMR interpretation. A detailed description of the reporting of NMR data in the form of peaklists can be found in the publication "Citation of NMR Peaklist Data within Patent Applications" (cf. http://www.researchdisclosure.com/searching-disclosures, Research Disclosure Database Number 605005, 2014, 01 Aug 2014). In the peak picking routine, as described in the Research Disclosure Database Number 605005, the parameter "MinimumHeight" can be adjusted between 1% and 4%. However, depending on the chemical structure and/or depending on the concentration of the measured compound it may be reasonable to set the parameter "MinimumHeight" <1%.

### HPLC methods

Method a (HPLC method 1 for pentaerythritol tetratosylate)

| | |
|---|---|
| **Column** | YMC Triart C18 (3.0 x 100 mm, 3 μm) |
| **Mobile phase** | A: 0.1% formic acid in water B：acetonitrile |
| **Flow** | 1.2 mL/min |
| **Injection** | 10 μL |
| **Sample solvent** | acetonitrile |
| **Column temperature** | 40 °C |
| **Gradient program** | <table><tr><td>Time</td><td>0</td><td>1</td><td>9</td><td>11</td></tr><tr><td>A%</td><td>98</td><td>98</td><td>20</td><td>20</td></tr><tr><td>B%</td><td>2</td><td>2</td><td>80</td><td>80</td></tr></table> |
| **Running time** | 11 min |
| **Detector** | UV-dectection, wavelength 198 nm |

Method b (HPLC method 2 for pentaerythritol tetratosylate)

| | |
|---|---|
| **Instrument** | High Performance Liquid Chromatograph equipped with variable wavelength UV detector and a suitable data handling system. |
| **Column** | Waters X-Select CSH C18 (150 mm × 4.6 mm, 3.5 μm) |
| **Mobile phase** | A: 0.1% orthophosphoric acid in water/Acetonitrile – 80/20 (%v/v)<br>B：0.1% orthophosphoric acid in water/Acetonitrile – 20/80 (%v/v) |
| **Flow** | 1.2 mL/min |
| **Injection** | 10 μL |
| **Sample solvent** | acetonitrile |
| **Sample concentration** | ~0.15 mg/mL |
| **Column temperature** | 40 °C |
| **Sample temperature** | 25 °C |
| **Gradient program** | <table><tr><td>Time</td><td>0</td><td>25</td><td>30</td><td>33</td><td>40</td></tr><tr><td>A%</td><td>80</td><td>10</td><td>10</td><td>80</td><td>80</td></tr><tr><td>B%</td><td>20</td><td>90</td><td>90</td><td>20</td><td>20</td></tr></table> |
| **Running time** | 40 min |
| **Detector** | UV detector, 225 nm |

Method c (HPLC/Assay 1 for Tetrabenzyl neopentyltetraamine hydrochloride)

| | |
|---|---|
| **Instrument** | High Performance Liquid Chromatograph equipped with variable wavelength UV detector and a suitable data handling system. |
| **Column** | Waters X-Select CSH C18 (150 mm × 4.6 mm, 3.5 μm) |

| | |
|---|---|
| **Mobile phase** | A: 0.1% orthophosphoric acid in water; B：acetonitrile |
| **Flow** | 0.6 mL/min |
| **Injection** | 10 µL |
| **Sample solvent** | Water/Acetonitrile/Methanol – 50/25/25 (%v/v/v) |
| **Sample concentration** | ~0.25 mg/mL |
| **Column temperature** | 15 °C |
| **Sample temperature** | 10 °C |
| **Gradient program** | Time: 0, 25, 30, 33, 40<br>A%: 92, 20, 20, 92, 92<br>B%: 08, 80, 80, 08, 08 |
| **Running time** | 40 min |
| **Detector** | UV detector, 210 nm |

Method d (HPLC method 2 for neopentyltetraamine hydrochloride, neopentyltetraamine acetate)

| | |
|---|---|
| **Instrument** | High Performance Liquid Chromatograph equipped with CAD detector and a suitable data handling system. |
| **Column** | Fortis H2O C18 (100 mm × 4.6 mm, 1.7 µm) |
| **Mobile phase** | A: 0.2% Pentafluoro propionic anhydride in Water; B：0.2% Pentafluoro propionic anhydride in Acetonitrile |
| **Flow** | 0.5 mL/min |
| **Injection** | 10 µL |
| **Sample solvent** | water |
| **Sample concentration** | 5.0 mg/mL |
| **Column temperature** | 20 °C |
| **Sample temperature** | 23 °C |
| **Gradient program** | Time: 0, 2.0, 15, 24, 28, 30, 35<br>A%: 90, 90, 75, 30, 30, 90, 90<br>B%: 10, 10, 25, 70, 70, 10, 10 |
| **Running time** | 35 min |
| **Detector** | CAD detector |
| **Evaporation temperature** | 50 °C |
| **Data collection rate** | 5 Hz |
| **Filter** | 10 s |

Method e (HPLC method 1 for purity of neopentyltetraamine hydrochloride)

| | |
|---|---|
| **Column** | YMC Pack Pro C4 (4.6 x 150 mm, 3 μm) |
| **Mobile phase** | A: 0.1% heptafluorbutyric acid in water B：0.1% heptafluorbutyric acid in acetonitrile |

| | |
|---|---|
| **Flow** | 1.0 mL/min |
| **Injection** | 5 μL |
| **Sample solvent** | water |
| **Sample concentration** | ~5.0 mg/mL |
| **Column temperature** | 20 °C |
| **Gradient program** | <table><tr><td>Time</td><td>0</td><td>2</td><td>15</td><td>24</td><td>25</td><td>30</td></tr><tr><td>A%</td><td>85</td><td>85</td><td>73</td><td>45</td><td>85</td><td>85</td></tr><tr><td>B%</td><td>15</td><td>15</td><td>27</td><td>55</td><td>15</td><td>15</td></tr></table> |
| **Running time** | 30 min |
| **Evaporating temperature:** | 50 °C |
| **Detector** | CAD detector |
| **Date collection rate** | 5 Hz |
| **Filter** | 10s |

Method f (HPLC method 2 for purity of tetrabenzyl neopentyltetraamine hydrochloride)

| | |
|---|---|
| **Column** | Agilent Zorbax Eclipse Plus C18 (3.0 x 100 mm, 3.5 μm) |
| **Mobile phase** | A: 0.1% trifluoro acetic acid in water B: acetonitrile |
| **Flow** | 1.5 mL/min |
| **Injection** | 5 μL |
| **Sample solvent** | Acetonitrile : water (9:1) |
| **Sample concentration** | ~1.0 mg/mL |
| **Column temperature** | 20 °C |
| **Gradient program** | <table><tr><td>Time [min]</td><td>0</td><td>7</td><td>10</td></tr><tr><td>A%</td><td>90</td><td>30</td><td>30</td></tr><tr><td>B%</td><td>10</td><td>70</td><td>70</td></tr></table> |
| **Running time** | 10 min |
| **Detector** | UV-dectection, wavelength 215 nm |
| **Date collection rate** | 5 Hz |
| **Filter** | 10s |

Method g (HPLC method 2 for purity neopentyltetraamine hydrochloride)

| | |
|---|---|
| **Column** | Cogent, Diamond Hydride (4.6 × 150 mm, 4 μm) |
| **Mobile phase** | A: 120 mM Ammoniumformiat pH 3 B: acetonitrile |
| **Flow** | 1.0 mL/min |
| **Injection** | 5 μL |
| **Sample solvent** | Water: acetonitrile 1:1 |
| **Sample concentration** | ~3.0 mg/mL |
| **Column temperature** | 40 °C |
| **Gradient program** | Isocratic; 30 % A + 70 % B |
| **Running time** | 15 min |
| **Detector** | ELSD; Gas pressure = 25 PSI, Gain = 500, time constant = 0.4 s, Nebulizer set point: 75 %, drift tube set point = 70 °C |

**Method h: (Ion Chromatography method for chloride and acetate)**

| | |
|---|---|
| Test Procedure | A: Used for Acetate |
| Test Procedure B: | Used for Chloride |
| Instrument (A and B): | Ion Chromatograph, e.g.:<br>Thermo Dionex ICS 5000+ or<br>Metrohm 940 Professional IC Vario |
| Eluent (gradient, A): | ~29 mmol/L NaOH |
| Eluent (isocratic, B): | 4.6 mM $Na_2CO_3$, 1.3 mM $NaHCO_3$, 5 % Acetone |
| Suppressor solution (A and B): | 0.5 M $H_2SO_4$ |
| Flow rate (A or B): | 1.8 mL/min or 0.7 mL/min |
| Column (A): | IonPac AS11 (250 x 4 mm) with pre-column (IonPac AS11, 50 x 4 mm) |
| Column (B): | Metrosep A Supp 5 (150 mm, particle size: 4.0) with pre-column (Metrosep A Supp 5 Guard) |
| Sample preparation (A): | 0.1 % (e.g. 25 mg/25 mL) in 20% MeOH |
| Sample preparation (B): | 0.1 % (e.g. 25 mg/25 mL) in water |
| Injection vol. (A or B): | 50 μL or 20 μL |
| Detection (A and B): | Conductivity |

**Assay determination**

**Method A-a (Assay determination for final product)**

The assay is determined as described below:

NPTA free base (%) = 100.0-TV-ROI-HCl-Sum VU

1,3-diamino-2,2-bis(aminomethyl)propane tetrahydrochloride,

in dry substance (%) = [NPTA free base(%)/0.4755]*[100/(100-TV)]

| | |
|---|---|
| TV | = Loss on drying [%] |
| ROI | = Residue on Ignition [%] |
| HCl | = assay chloride ×1.0284 |
| Sum VU | = Summe sidecomponents in HPLC [%] |
| 0.4755 | = Calculation Factor 1,3-diamino-2,2-bis(aminomethyl)propane tetrahydrochloride to NPTA free Base |
| | ($M_W$ NPTA free Base / $M_W$ 2,2bis(aminomethyl)propane-1,3-diamine tetrahydrochloride = 132.21 / 278.05) |
| 1.0284 | = Calculation factor chloride to hydrochloride |
| | ($M_W$ HCl / $M_W$Cl- = 36.461 / 35.453) |

**Method A-b (Assay determination by qNMR)**

Dissolve 1,3-diamino-2,2-bis(aminomethyl)propane tetrahydrochloride or 1,3-diamino-2,2-bis(aminomethyl) propane tetraacetic acid salt at a suitable concentration in $D_2O$. Use suitable measuring parameters (temperature, relaxation delay, pulse angle, acquisition time, spectral width, number of scans) and quantify by internal standard method using a suitable internal standard soluble in $D_2O$.

Use a spectrometer frequency of at least 400 MHz.

Take into account the proton numbers for the evaluated signals and the molecular weights of the internal standard and 2,2-bis(aminomethyl)propane-1,3-diamine tetrahydrochloride.

**Loss on Drying**

**Method L**

according to Ph.Eur. 2.4.32

| | |
|---|---|
| Test procedure | 1.000 (0.900 - 1.100) g sample are placed in a pre-weight weighing glass that has previously been dried at (105 ± 2) °C a drying cabinet for at least 30 minutes. |
| | The weighing glass is heated to (105 ± 2) °C for 2 h in a drying cabinet and subsequently allowed to cool to room temperature in a desiccator with silica gel as a drying agent. Thereafter, the weighing glass is weighed. |
| Calculation | Loss on drying [%]<br>= [[m[1] - (m[3] - m[2])] * 100]/m[1] |
| | m[1] = Sample mass [g] |

(continued)

| | |
|---|---|
| m[2] | = Sample glas [g, Tara] |
| m[3] | = Residue after drying with sample glas [g] |

**Residue on Ignition**

| | |
|---|---|
| Test procedure | 1.0000 (0.9000 - 1.1000) g sample are placed in a pre-weight porcelain crucible that has previously been glowed at (600 ± 25) C in a muffle overnight. The sample is charged in a pre-asher at lowest possible temperature. Then, the residue is chared and glowed at (600 ± 25) C in a muffle overnight. |
| | The porcelain crucible is allowed to cool at ambient atmosphere for 5 min, subsequently placed in a dessicator with drying agent and further allowed to cool to room temperature. Thereafter, the porcelain crucible is weighed. |
| Calculation | Residue on ignition [%]<br>= [(m[3] - m[2])*100]/m[1] |
| | m[1] = Sample mass [g]<br>m[2] = porcelain crucible [g, Tara]<br>m[3] = Residue on ignition with porcelain crucible [g] |

**Palladium content**

Pd content was determined according to Ph.Eur. 2.4.20 and Ph.Eur. 2.2.58 utilizing ICP-MS after dissolving the sample under aqueous acidic conditions and heat in a microwave oven.

**Chloride content via titration**

Chloride content was determined by titration | potentiometric titration according to Ph.Eur. 2.2.20, USP <541>

| | |
|---|---|
| Reagents | Aqueous silver(I) nitrate solution (0.1 M), water, aqueous nitric acid (12.5 wt-%) |
| Apparatus | Titration apparatus, i.e. Metrohm or Mettler |
| Electrode | i.e. Ag/Ag electrode |
| Burette | Piston burette with suitable volume, e.g. 20 mL |
| End of titration | Automized via Titropocessor. |
| Calculation | Assay chloride in %<br>= ( V*T*35.45*0.1*100)/(m*1000) |
| | T = Titer silver(I) nitrate solution 0.1M<br>V = Consumption silver(I) nitrate solution [ml]<br>m = Sample weight [g]<br>35.45 = Mass analytic equivalent = molecular mass chloride [g/mol] |
| | 1 mL silver(I) nitrate solution 0.1M equals 3.545 mg chloride. |

**Water content determination**

*Water content (method a):*

Instrument: 870 KF-Titrino Plus, Metrohm AG, Herisau, Switzerland; solvent: Hydranal® solvent for the volumetric determination of water content according to Karl-Fischer; titrant: Hydranal®-titrant 5.

**Instrumental set-up for the XRPD measurements**

X-ray powder diffraction (XRPD) data were recorded on a STOE STADI P diffractometer using monochromatized $CuK_{\alpha 1}$-radiation, a position sensitive detector, at generator settings of 40 kV and 40 mA. The samples were collected in transition mode, being prepared as a thin layer between two foils. The scanning range was between 2 ° and 40 ° 2 theta with a 0.5° step at 15 sec/step.Instrumental set-up for the IR measurements

IR measurements were performed with a Bruker alpha spectrometer in the attenuated total reflectance (ATR) geometry. No sample preparation was performed, and each individual measurement consisted of 32 scans.

**Instrumental set-up for the Raman measurements**

Raman measurements were performed with a Bruker MultiRAM spectrometer. No sample preparation was performed, and each individual measurement consisted of 64 scans using a laser power of 300 mW.

**Example 1 - Synthesis of Neopentyltetraamine Hydrochloride**

**Step 1: Pentaerythritol-tetratosylate**

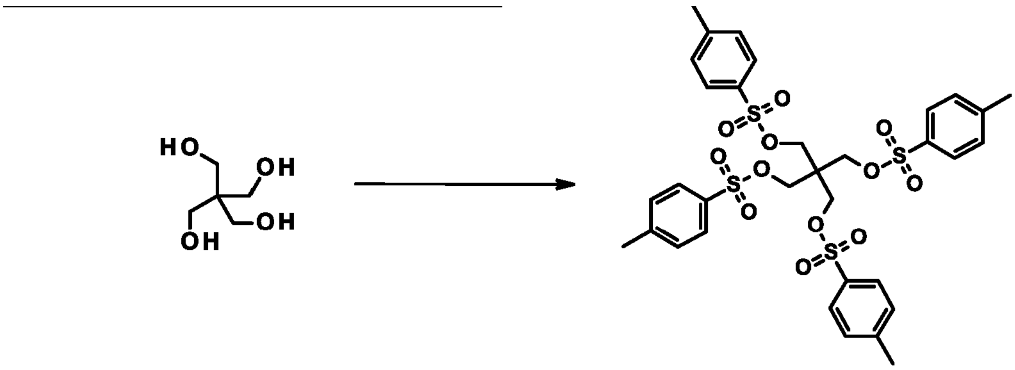

MW: 136,15 g/mol
$C_5H_{12}O_4$

MW: 752,89 g/mol
$C_{33}H_{36}O_{12}S_4$

Pentaerythritol (1.00 kg) and pyridine (9.88 kg) were placed in a 40 L reactor and the light brown solution was agitated at 20 °C. *para*-Toluenesulfonylchloride (7.00 kg, 5 eq) was added portion wise (ca 1 kg portions). The thin suspension was heated to 30 °C and stirred for 24 h. after which the suspension was cooled to 15 °C. Then, over the course of 10 min, the suspension was transferred onto 3 °C cold water (22.2 kg). After complete transfer, the suspension (12 °C) was heated to 15 °C and stirred for 20 min at this temperature. The solids were then isolated *via* filtration and washed with water (3 × 11.1 kg) and ethanol (5.92 kg). The material was then dissolved in dichloromethane (29.46 kg). Then, ca 11 L of dichloromethane were distilled off at 60 °C and the light-yellow solution was cooled to 20 °C. Ethanol (22.1 kg) was added over the course of 30 mins. The resulting white suspension was stirred at 20°C overnight. Solids were then isolated *via* filtration and washed with ethanol (3.95 kg). The white solid was dried under vacuum at 50 °C to constant mass to give pentaerythritol-tetratosylate as a free-flowing, white powder (4.77 kg, 86% yield)

| | |
|---|---|
| HPLC (method a): | $t_R$ = 9.382 min |
| Purity (method a): | 97.8 Area-% |

The following Examples have been carried out similarly as described above:

| Example | Scale pentaerythritol [kg] | Yield tetratosylate [kg \| %] | Purity [Area-%, method b] |
|---|---|---|---|
| 1a§ | 103.9 kg | 518.4 kg \| 90% | 99.7 |
| 1b§ | 118.0 kg | 596.6 kg \| 92% | 99.6 |
| § washing of the intermediate solid and final precipation was carried out with methanol instead of ethanol. | | | |

**Step 2 : Pentaerythritol-tetrabenzylamine tetrahydrochloride**

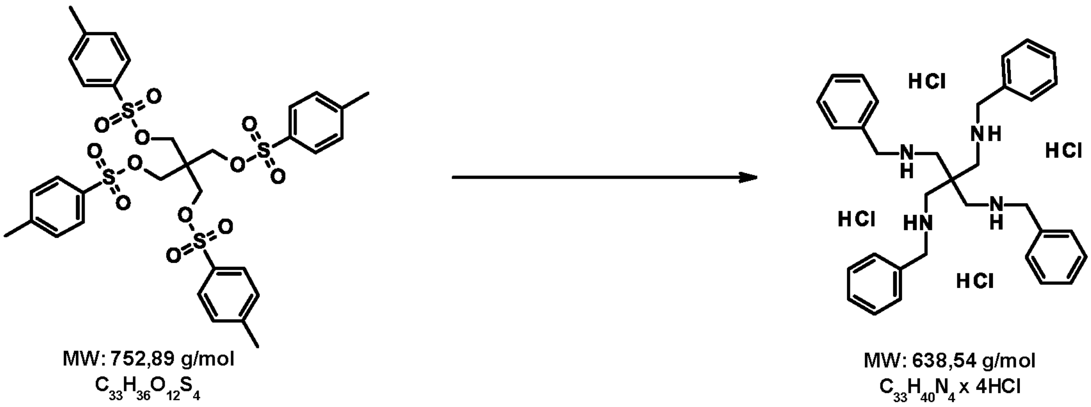

MW: 752,89 g/mol
$C_{33}H_{36}O_{12}S_4$

MW: 638,54 g/mol
$C_{33}H_{40}N_4$ x 4HCl

In a 60 L reactor, a suspension of pentaerythritol tetratosylate (0.666 kg) and benzyl amine (5.692 kg) were stirred under an inert atmosphere. The reaction mixture was heated to 140 °C over the course of 1 h, kept at 140 °C for 2 h, and then cooled to 100 °C. Another portion of tosylate was added (0.666 kg), and the reaction heated to 140 °C, stirred for 2 h, cooled to 100 °C, before the last portion of tosylate (0.668 kg) was added, the mixture heated to 140 °C and stirred at that temperature for an additional 6 h. The mixture was the cooled to 50 °C and stirred overnight. Then, toluene (13.87 kg) was added, the resulting suspension cooled to 20 °C and stirred for 15 min. Solids were removed by filtration and the filter cake washed with toluene (6.07 kg). To the filtrate was added water (16.0 kg) and the mixture was cooled to 10-15 °C. The pH was adjusted to 8.0 using concentrated hydrochloric acid (ca 3.0 kg) and stirred for 10 min at 15 °C. The mixture was transferred to a phase separator and the aqueous phase (ca 24 L) was discarded. The organic phase was washed once more with water (20 kg). The organic fraction (ca 22 L) was concentrated to ca. 7 L (60 °C, 100 mbar, ca. 14 L of distillate collected).

Ethanol (18.9 kg) was added to the toluene solution and the solution heated to 60 °C. Over the course of 20 min, concentrated aqueous hydrochloric acid (2.094 kg) was added and the suspension stirred at 60 °C for 1h, then cooled to 20 °C and stirred for an additional 2 h. The solids were collected *via* filtration and dried at 55 °C under vacuum to yield tetrabenzylneopentyltetraamine tetrahydrochloride (1.565 kg, 92%) as a white solid.

| | |
|---|---|
| HPLC (method f): | $t_R$ = 4.362 min |
| Purity (method f): | 97.9 Area-% |
| $^1$H-NMR ($D_2O$): | δ = 7.50 - 7.38 (m, 20H), 4.16 (s, 8H), 3.33 (s, 8H) ppm. |
| $^{13}$C-NMR ($D_2O$): | δ = 131.42, 129.75, 129.57, 129.23, 52.87, 49.47, 38.13 ppm. |
| HRMS (+ESI): | calc. for $C_{33}H_{41}N_4^+$: 493.3326; found: 493.3328 |

**IR (Band maxima, cm-1):**

| NPTA Tetrabenzyl x4HCl |
|---|
| 449 |

(continued)

| NPTA Tetrabenzyl x4HCl |
|---|
| 497 |
| 504 |
| 583 |
| 602 |
| 612 |
| 695 |
| 752 |
| 767 |
| 814 |
| 850 |
| 892 |
| 917 |
| 952 |
| 1037 |
| 1077 |
| 1218 |
| 1344 |
| 1423 |
| 1448 |
| 1458 |
| 1499 |
| 1543 |
| 1552 |
| 2360 |
| 2616 |
| 2722 |
| 2931 |
| 2982 |

Raman (Band maxima, cm-1):

| NPTA Tetrabenzyl x4HCl |
|---|
| 209 |
| 291 |
| 323 |
| 407 |
| 451 |
| 480 |
| 502 |
| 620 |
| 749 |
| 758 |
| 784 |
| 866 |
| 916 |
| 948 |
| 970 |

(continued)

| NPTA Tetrabenzyl x4HCl |
|---|
| 1005 |
| 1037 |
| 1051 |
| 1062 |
| 1160 |
| 1188 |
| 1221 |
| 1343 |
| 1426 |
| 1447 |
| 1462 |
| 1480 |
| 1588 |
| 1609 |
| 2890 |
| 2966 |
| 2990 |
| 3008 |
| 3064 |

**XRPD (Diffraction angle 2θ, °):**

| NPTA Tetrabenzyl x4HCl |
|---|
| 6.2 |
| 7.5 |
| 9.0 |
| 10.4 |
| 10.6 |
| 10.9 |
| 12.9 |
| 13.4 |
| 14.2 |
| 14.5 |
| 15.3 |
| 15.7 |
| 15.9 |
| 16.3 |
| 17.4 |
| 17.8 |
| 18.3 |
| 18.9 |
| 19.2 |
| 20.0 |
| 20.2 |
| 20.5 |
| 20.8 |
| 21.0 |

(continued)

| NPTA Tetrabenzyl x4HCl |
|---|
| 21.7 |
| 22.1 |
| 22.5 |
| 22.8 |
| 23.2 |
| 23.6 |
| 24.2 |
| 24.7 |
| 26.1 |
| 26.2 |
| 26.6 |
| 26.9 |
| 27.2 |
| 27.7 |
| 28.0 |
| 28.2 |
| 28.4 |
| 28.6 |
| 29.2 |
| 29.9 |
| 30.5 |
| 30.7 |
| 30.9 |
| 31.5 |
| 31.6 |
| 32.3 |
| 32.7 |
| 33.0 |
| 33.2 |
| 33.3 |
| 33.7 |
| 34.0 |
| 34.3 |
| 34.7 |
| 35.4 |
| 35.9 |
| 36.1 |
| 36.4 |
| 36.5 |
| 37.1 |
| 37.4 |
| 37.7 |
| 37.9 |
| 38.2 |
| 38.9 |
| 39.4 |

The following Examples have been carried out similarly as described above:

| Example | Scale pentaerythritol tetratosylate [kg] | Yield tetrabenzylamine [kg \| %] | Purity [Area-%, method c] | Assay [wt-%, method c] |
|---|---|---|---|---|
| 2a§ | 275.0 kg | 197.9 kg \| 85% | 98.3 | 99.5 |
| 2b§ | 275.0 kg | 220.6 kg \| 95% | 98.8 | 97.8 |
| 2c§ | 295.0 kg | 235.7 \| 94% | 99.0 | 96.3 |

§ methanol instead of ethanol was added for the crystallization of the hydrochloride salt. The portionwise addition of pentaerythritol tetratosylate was carried out while maintaining an internal temperature of 140 °C

**Step 3: Neopentyltetraamine Acetate**

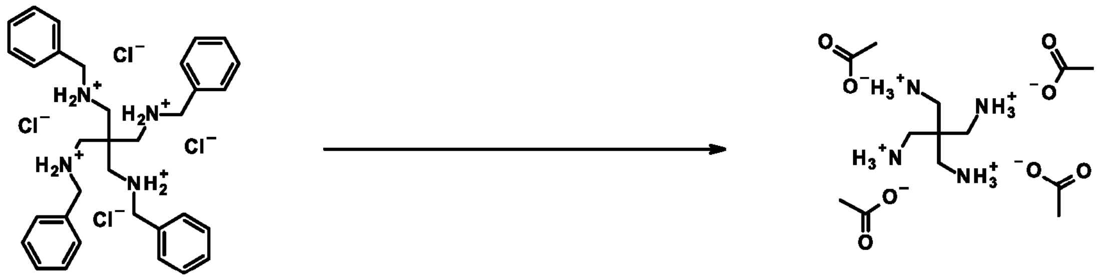

MW: 638,54 g/mol
$C_{33}H_{40}N_4$ x 4HCl

MW: 372,42 g/mol
$C_5H_{20}N_4.4C_2H_3O_2$

Tetrabenzyl neopentyltetraamine hydrochloride (1 kg) was suspended in MTBE (7.1 kg) under an inert atmosphere in a 60 L reactor. Aqueous sodium hydroxide solution (7.4%, 8.06 kg) was added and the mixture heated to 37 °C. Stirring for 4 h provided a biphasic mixture. The aqueous phase was separated (ca 8 L) and organic phase stirred for 15 min with additional aqueous sodium hydroxide solution (7.4%, 8.06 kg), the aqueous phase discarded, and the organic portion stirred once more for 15 min with water (7.4 kg). The organic portion was separated and concentrated from ca 9.8 L to a volume of approx. 4 L (50 °C mantle, 300 mbar, 27 °C pot temperature at endpoint). Isopropanol (9.85 kg) was added and the solution was concentrated to ca 5 L (60 °C mantle, 100 mbar, 35 °C pot temperature at endpoint). The solution (2.93 kg) was then transferred into a barrel, and isopropanol was added to give a total weight of the solution of 7.86 kg.

An inertized 20L autoclave was charged with 20% Pd CP MUR catalyst (74 g, Pearlman type, BASF SE), water (1.00 kg) and acetic acid (360 g), followed by additional water (430 g). The vessel was inertized, and the isopropanolic solution of the free base (7.68 kg) was pumped into the reactor, and the transfer completed with additional isopropanol (3.00 kg).

The hydrogenation was then initiated by pressurizing the autoclave with hydrogen (25 °C, 3 bar) and the hydrogen pressure was kept constant for 20 h (hydrogen uptake ceased after 19 h). Then, the suspension was pumped onto a filter to remove the catalyst and the filter washed with isopropanol.

The filtrate (ca 16 L) was concentrated to ca ¼ volume (12 L distillate, 60 °C, vacuum). Then, isopropanol (12.14 kg) was added and the solution was again concentrated under reduced pressure (6 L distillate, 60 °C, vacuum), and then cooled to 45 °C. The solution was analyzed for water content (KF, ca. 2.5%) and residual toluene (GC-headspace, 0.03%).

Then, acetic acid (282 g, 3 eq) was added, followed by seed crystals (neopentyltetraamine acetate, 5 g). The solution was cooled to 20 °C over the course of 20 min, was stirred at this temperature for 2 h, then cooled to 5 °C, stirred for another 2 h , then cooled to -5 °C and stirred at this temperature for 18 h. The solids were isolated by filtration, suction dried for 30 min and then dried at 50 °C under vacuum to yield neopentyltetraamine acetate (560 g, 96% of theory) as a white, crystalline solid.

Analytics:

| | |
|---|---|
| $^1$H-NMR ($D_2O$): | $\delta$ = 3.21 (s, 8H), 1,96 (s, 12H) ppm. |
| $^{13}$C-NMR ($D_2O$): | $\delta$ = 180.27, 41.27, 37.12, 22.52 ppm. |

(continued)

| | |
|---|---|
| Purity by NMR: | >95% |
| Assay (method A-b): | 96.7% |
| HRMS (+ESI): | calc. for $C_5H_{17}N_4^+$: 133.1448; found: 133.1450 |

**IR (Band maxima, cm-1):**

| NPTA Acetate |
|---|
| 412 |
| 465 |
| 558 |
| 618 |
| 655 |
| 744 |
| 848 |
| 923 |
| 982 |
| 1016 |
| 1046 |
| 1090 |
| 1133 |
| 1167 |
| 1332 |
| 1403 |
| 1486 |
| 1516 |
| 1635 |

**Raman (Band maxima, cm-1):**

| NPTA Acetate |
|---|
| 334 |
| 410 |
| 471 |
| 661 |
| 745 |
| 848 |
| 926 |
| 985 |
| 1034 |
| 1047 |
| 1200 |
| 1351 |
| 1412 |
| 1491 |
| 2929 |

(continued)

| NPTA Acetate |
|---|
| 2986 |

**XRPD (Diffraction angle 2θ, °):**

| NPTA Acetate |
|---|
| 9.8 |
| 10.3 |
| 11.4 |
| 12.1 |
| 12.4 |
| 13.1 |
| 13.4 |
| 15.1 |
| 15.6 |
| 17.4 |
| 17.9 |
| 18.1 |
| 18.8 |
| 19.8 |
| 20.6 |
| 21.2 |
| 21.6 |
| 22.4 |
| 22.9 |
| 23.3 |
| 24.4 |
| 25.0 |
| 25.6 |
| 26.1 |
| 26.4 |
| 27.0 |
| 27.7 |
| 28.4 |
| 28.8 |
| 29.2 |
| 29.6 |
| 29.9 |
| 30.4 |
| 30.6 |
| 30.9 |
| 31.6 |
| 31.8 |
| 32.1 |
| 32.4 |
| 33.7 |
| 34.1 |
| 34.8 |

(continued)

| NPTA Acetate |
|---|
| 35.2 |
| 35.6 |
| 36.3 |
| 36.6 |
| 36.7 |
| 37.0 |
| 37.3 |
| 38.0 |
| 38.4 |
| 38.8 |
| 39.3 |

The following Examples have been carried out similarly as described above:

| Example | Scale Tetrabenzyl-neopentyl-tetramine hydrochloride [kg] | Yield neopentyltetraamine tetraacetate [kg \| %] | Sum of all impurites [wt-%\| method d] | Palladium content [ppm] |
|---|---|---|---|---|
| 3a | 137.7 kg | 70.4 kg \| 88% | 0.19 | 1 |
| 3b | 142.0 kg | 72.0 kg \| 87% | 0.28 | <1 |
| 3c | 142.0 kg | 70.5 kg \| 85% | 0.26 | 1 |

**Step 4: Neopentyltetraamine Tetrahydrochloride**

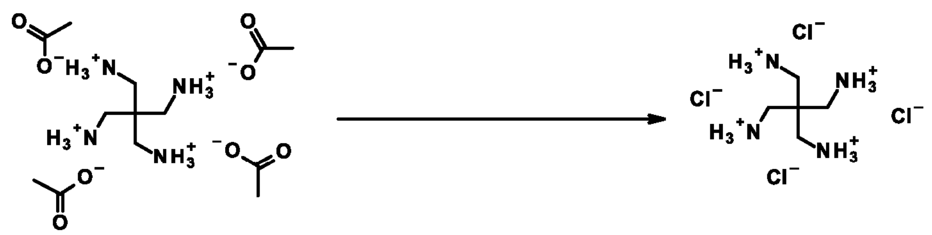

MW: 372,42 g/mol
$C_5H_{20}N_4.4C_2H_3O_2$

MW 278,05 g/mol
$C_5H_{20}N_4.4Cl$

Neopentyltetraamine tetraacetate (300 g) was dissolved in water (750 mL). Over the course of 90 min, while keeping the temperature below 25 °C, concentrated hydrochloric acid (337 mL, 5 eq) was added, which caused rapid precipitation of solids. Then, isopropanol (2250 mL) was added over the course of 30 min at room temperature (to achieve a v/v of 1:3 of water and isopropanol). The suspension was heated to 50 °C and was stirred at this temperature for 1 h. The suspension was cooled to 20 °C over the course of 1 h, and stirred at this temperature for an additional 1 h. The solids were isolated by filtration, washed twice with a 4:1 isopropanol/water mixture (2 × 200 mL) and dried at 50 °C under vacuum yielding 219 g of a white solid. The solid was then suspended in a 1:3 mixture of water and isopropanol (v/v), agitated at 50 °C for 1 h, cooled to 20 °C over the course of 1 h and stirred an additional 1 h at this temperature. The solids were isolated by filtration, washed once with a 4:1 isopropanol/water mixture (300 mL) and dried at 70 °C under vacuum yielding a white, crystalline solid (213 g, 95.3%).

| | |
|---|---|
| Assay (method A-b): | 99.3 wt-% |
| Purity (method g): | >99.9% |
| $^1$H-NMR ($D_2O$): | $\delta$ = 3.47 (s, 8H) ppm. |
| $^{13}$C-NMR ($D_2O$): | $\delta$ = 38.70 ppm. (quarternary carbon not visible) |

(continued)

| | |
|---|---|
| HRMS (+ESI): | calc. for $C_5H_{17}N_4^+$: 133.1448; found: 133.1450 |
| Chloride content (Method h): | 49 wt-% |
| Acetate content (Method h): | <0.01 wt% |

The following examples has been carried out similarly as described above:

| Example | Scale Neopentyltetraamine tetraacetate | Yield 1,3-diamino-2,2-bis(aminomethyl) propane tetrahydrochloride [kg \| %] | Sum of all impurites [Ar-%\| method e] | Palladium content [ppm] | Assay in dry substance [wt-%, method A-a] |
|---|---|---|---|---|---|
| 4a | 125.0 kg | 71.3 kg \| 77% | < 0.05 | < 1 | 99.9 |
| 4b | 131.3 kg | 80.0 kg \| 82% | < 0.05 | < 1 | 99.8 |
| 4c | 121.0 kg | 70.8 kg \| 78% | < 0.05 | 1 | 99.6 |

## Claims

**1.** A process for the preparation of 2,2-bis(aminomethyl)propane-1,3-diamine comprising

(i) providing sulfonic ester-activated pentaerythritol,
(ii) reacting the sulfonic ester-activated pentaerythritol with a benzylic amine to yield a benzylic neopentyltetraamine,
(iii) removing the benzylic group from the benzylic neopentyltetraamine and
(iv) isolating 2,2-bis(aminomethyl)propane-1,3-diamine as a hydrochloride salt, preferably as a tetrahydrochloride salt.

**2.** The process according to claim 1, wherein step (i) comprises providing sulfonic ester-activated pentaerythritol by

(i-a) providing pentaerythritol and
(i-b) reacting pentaerythritol with a sulfonyl halide to sulfonic ester-activated pentaerythritol.

**3.** The process according to any of claims 1 to 2, wherein the sulfonic ester-activated pentaerythritol is pentaerythritol tetratosylate and the sulfonyl halide is p-toluenesulfonyl chloride.

**4.** The process according to any of claims 1 to 3, wherein the benzylic amine is selected from the group consisting of benzyl amine, 4-methoxybenzylamine and 3,4-dimethoxybenzylamine, preferably wherein the benzylic amine is benzyl amine.

**5.** The process according to any of claims 1 to 4, wherein the reaction in step (ii) is carried out at temperatures ranging of from 100 °C to 180 °C, preferably of from 120 °C to 160 °C, more preferably of from 130 to 150 °C and with a reaction time ranging of from 1 h to 48 h, prerably of from 4 h to 24 h, more preferably of from 6 h to 10 h.

**6.** The process according to any of claims 1 to 5, wherein the reaction in step (ii) is carried out in neat benzylic amine using 4 equivalents to 50 equivalents of the benzylic amine, preferably 10 equivalents to 40 equivalents of the benzylic amine, more preferably 15 equivalents to 25 equivalents of the benzylic amine.

**7.** The process according to any of claims 1 to 6, wherein step (iii) comprises removing the benzylic group from the benzylic neopentyltetraamine and optionally isolating 2,2-bis(aminomethyl)propane-1,3-diamine as an acetate salt, preferably as a tetraacetate salt and step (iv) comprises converting the acetate salt of 2,2-bis(aminomethyl) propane-1,3-diamine to its hydrochloride salt and isolating 2,2-bis(aminomethyl)propane-1,3-diamine as a hydrochloride salt, preferably as a tetrahydrochloride salt.

**8.** The process according to any of claims 1 to 6, wherein step (iii) comprises removing the benzylic group from the

benzylic neopentyltetraamine and isolating 2,2-bis(aminomethyl)propane-1,3-diamine as an acetate salt, preferably as a tetraacetate salt and step (iv) comprises converting the acetate salt of 2,2-bis(aminomethyl)propane-1,3-diamine to its hydrochloride salt and isolating 2,2-bis(aminomethyl)propane-1,3-diamine as a hydrochloride salt, preferably as a tetrahydrochloride salt.

**9.** The process according to any of claims 1 to 6, wherein step (iii) comprises removing the benzylic group from the benzylic neopentyltetraamine in the presence of acetic acid and isolating 2,2-bis(aminomethyl)propane-1,3-diamine as an acetate salt, preferably as a tetraacetate salt and step (iv) comprises converting the acetate salt of 2,2-bis(aminomethyl)propane-1,3-diamine to its hydrochloride salt and isolating 2,2-bis(aminomethyl)propane-1,3-diamine as a hydrochloride salt, preferably as a tetrahydrochloride salt.

**10.** The process according to any of claims 1 to 6, wherein step (iii) comprises removing the benzylic group from the benzylic neopentyltetraamine in a mixture consisting of two solvents, the first solvent being an alcohol, and the second solvent being water in the presence of acetic acid and isolating 2,2-bis(aminomethyl)propane-1,3-diamine as an acetate salt, preferably as a tetraacetate salt and step (iv) comprises converting the acetate salt of 2,2-bis(aminomethyl)propane-1,3-diamine to its hydrochloride salt and isolating 2,2-bis(aminomethyl)propane-1,3-diamine as a hydrochloride salt, preferably as a tetrahydrochloride salt.

**11.** The process according to any of claims 7 to 10, wherein step (iv) comprises converting the tetraacetate salt of 2,2-bis(aminomethyl)propane-1,3-diamine to its hydrochloride salt with hydrochloric acid in water, preferably using 4 to 10 equivalents of hydrochloric acid, more preferably using 5 equivalents of hydrochloric acid and isolating 2,2-bis(aminomethyl)propane-1,3-diamine as a hydrochloride salt, preferably as a tetrahydrochloride salt.

**12.** The process according to claim 11, wherein the isolation of 2,2-bis(aminomethyl)propane-1,3-diamine as a hydrochloride salt, preferably as a tetrahydrochloride salt comprises the addition of an organic solvent selected from methanol, ethanol, n-propanol, 2-propanol and n-butanol, preferably wherein the organic solvent is 2-propanol.

**13.** A crystalline tetraacetate salt of 2,2-bis(aminomethyl)propane-1,3-diamine **characterized by** having a powder X-ray diffractogram comprising reflections at 2-Theta angles of (9.8 ± 0.2)°, (11.4 ± 0.2)°, (20.6 ± 0.2)°, (22.4 ± 0.2)° and (25.0 ± 0.2)°, when measured at room temperature with Cu-Kalpha$_1$ radiation having a wavelength of 0.15419 nm.

**14.** A crystalline tetrahydrochloride salt of N,N'-dibenzyl-2,2-bis[(benzylamino)methyl]propane-1,3-diamine **characterized by** having a powder X-ray diffractogram comprising reflections at 2-Theta angles of (9.0 ± 0.2)°, (14.5 ± 0.2)°, (21.7 ± 0.2)°, (22.8 ± 0.2)° and (26.9 ± 0.2)°, when measured at room temperature with Cu-Kalpha$_1$ radiation having a wavelength of 0.15419 nm.

**15.** Use of a process according to any of claims 1 to 12 for the preparation of 2,2-bis(aminomethyl)propane-1,3-diamine, preferably as a tetrahydrochloride salt.

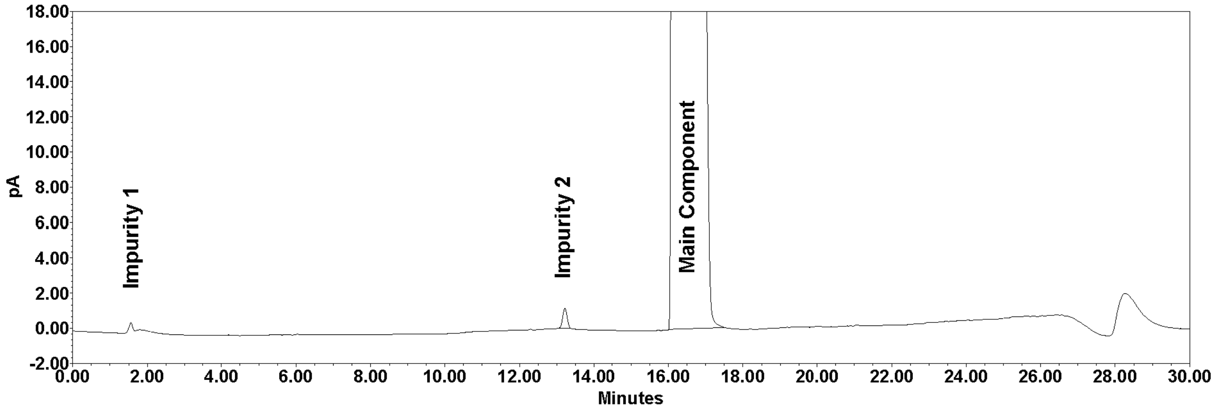


Figure 1

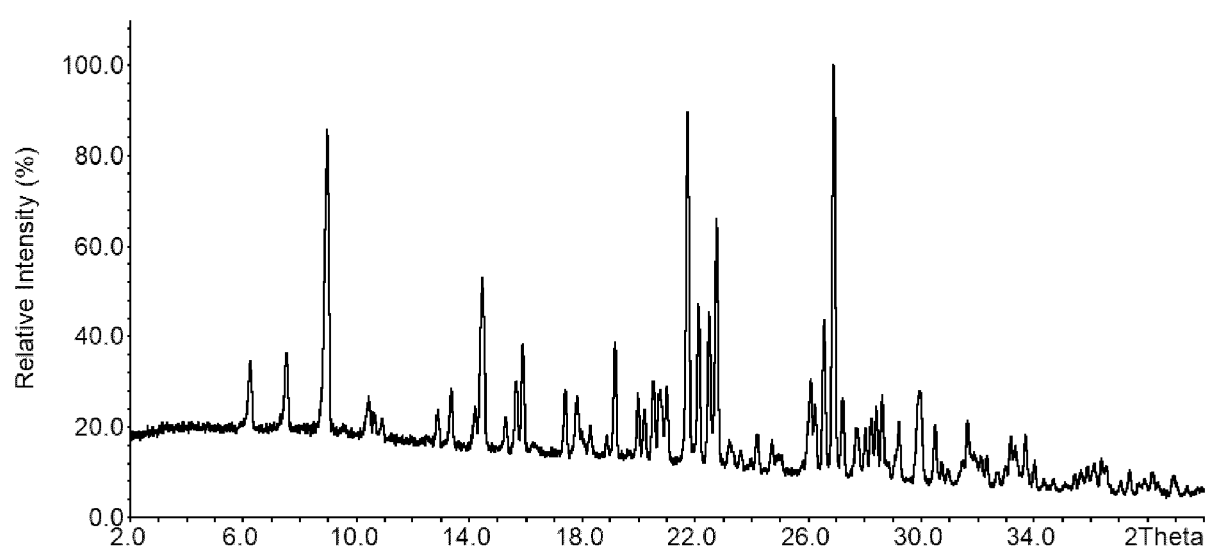


Figure 2

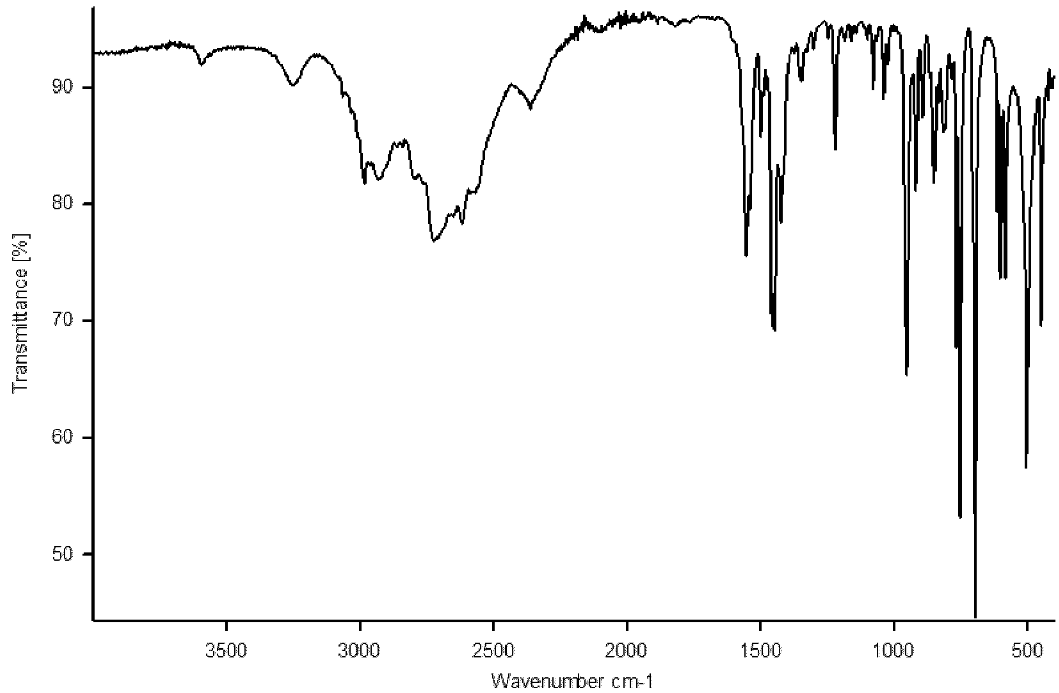


Figure 3

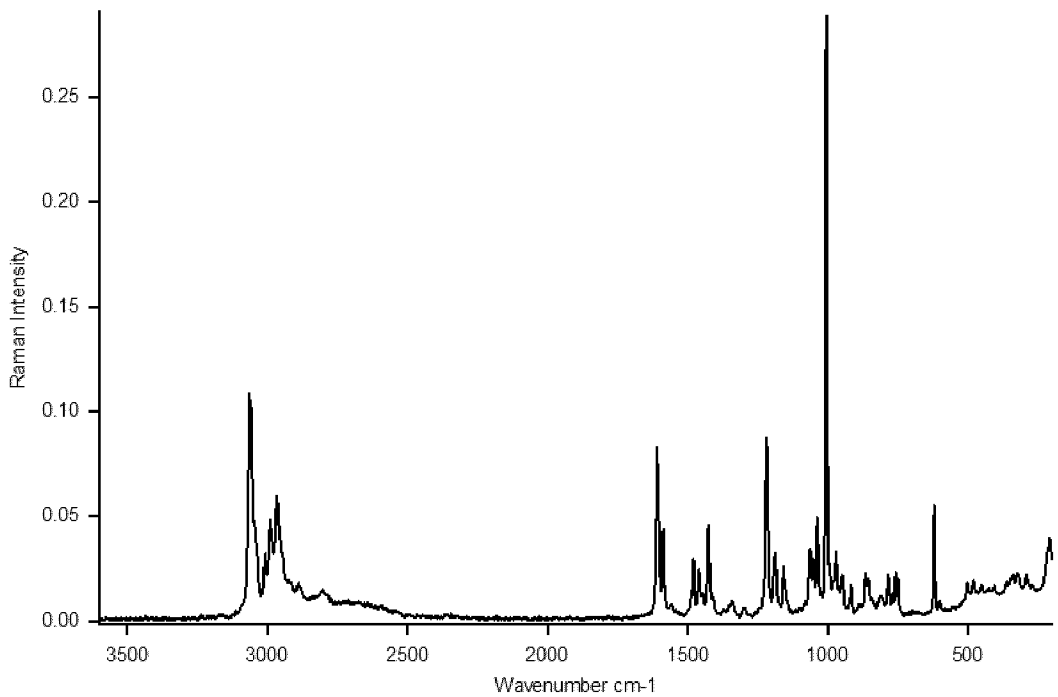


Figure 4

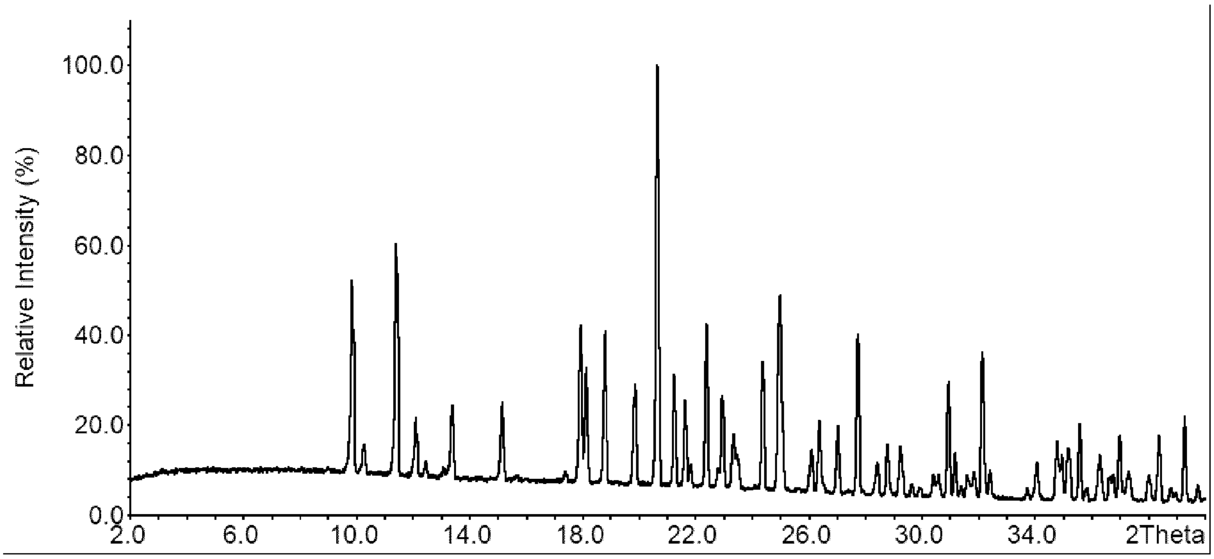


Figure 5

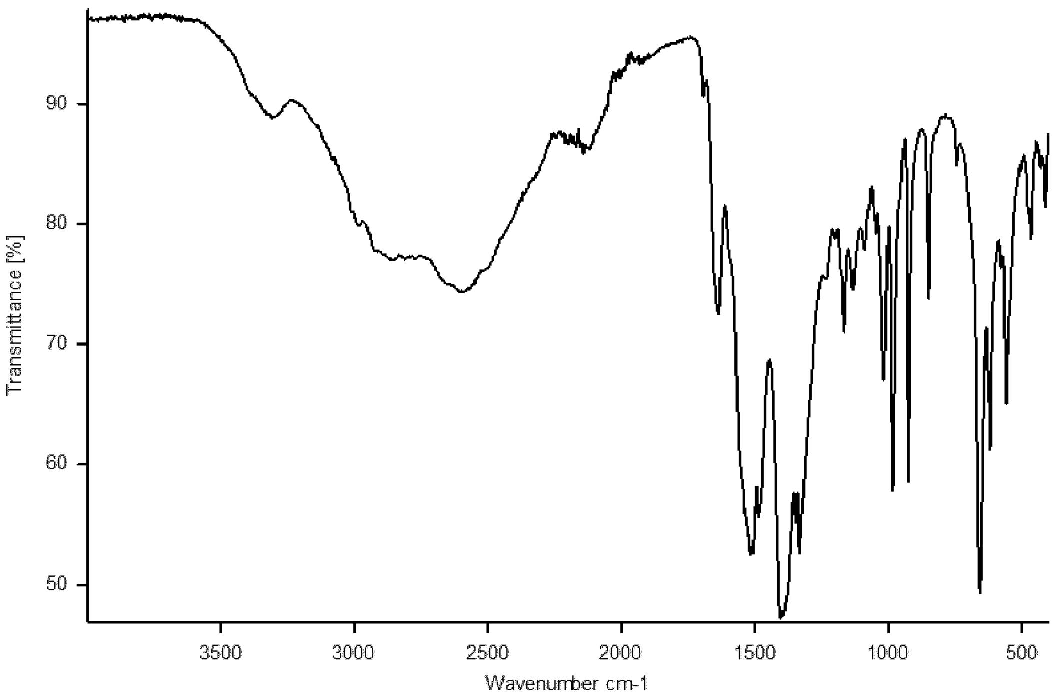


Figure 6

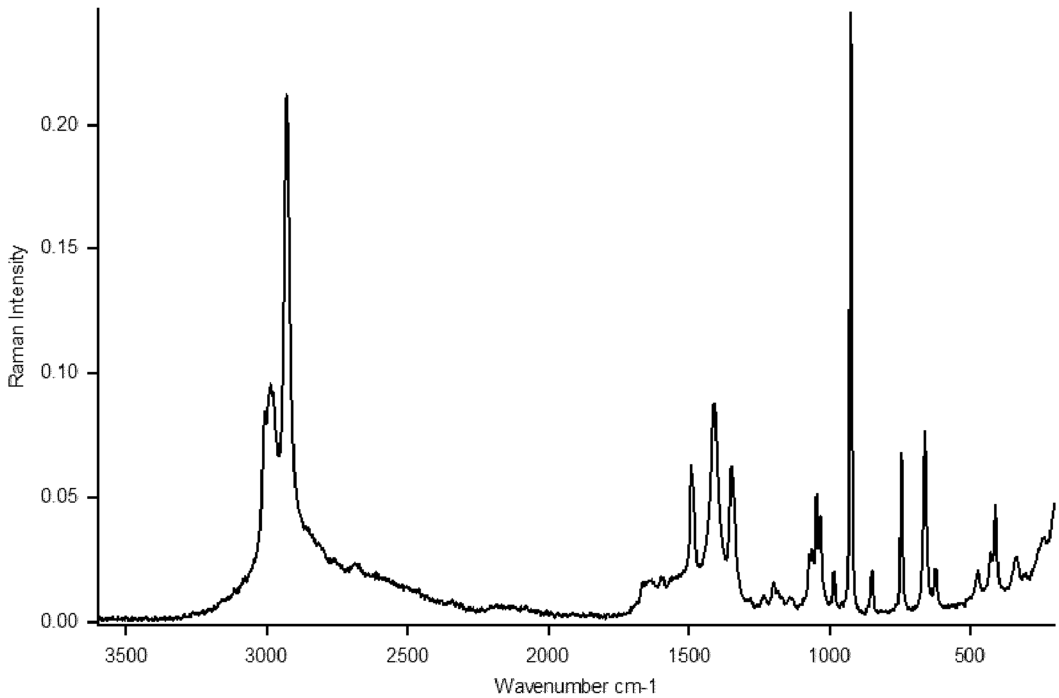
Raman Intensity
0.20
0.15
0.10
0.05
0.00
3500
3000
2500
2000
1500
1000
500
Wavenumber cm-1

Figure 7

Europäisches Patentamt
European Patent Office
Office européen des brevets

# EUROPEAN SEARCH REPORT

Application Number
EP 24 18 4512

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | DE ANDRADE SAULO F. ET AL: "An Efficient Synthesis of D-Galactose-Based Multivalent Neoglycoconjugates", J. BRAZ. CHEM. SOC., June 2012 (2012-06), pages 1062-1069, XP093238545, DOI: https://doi.org/10.1590/S0103-50532012000600010 Retrieved from the Internet: URL:https://www.scielo.br/j/jbchs/a/dmRQLY78wzBTkLWkNQvV3Zd/?format=pdf&lang=en [retrieved on 2025-12-31] * Scheme 2; compounds 11-12 * * page 1069, left-hand column * ----- | 1-15 | INV. C07C209/16 C07C209/62 C07C211/13 C07C303/28 |
| X | VAN ALPHEN J.: "On aliphatic polyamines VII", RECUEIL DES TRAVAUX CHIMIQUES DES PAYS-BAS, vol. 57, no. 3, 13 January 1938 (1938-01-13), pages 265-276, XP093238686, Amsterdam, NL ISSN: 0165-0513, DOI: 10.1002/recl.19380570304 * page 268 - page 269 * ----- | 14 | |
| | | | TECHNICAL FIELDS SEARCHED (IPC) |
| | | | C07C |

The present search report has been drawn up for all claims

1

| Place of search | Date of completion of the search | Examiner |
|---|---|---|
| The Hague | 14 January 2025 | Kövecs, Monika |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
..........................................................................................................
& : member of the same patent family, corresponding document

EPO FORM 1503 03.82 (P04C01)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2016193190 A
- WO 2024088910 A
- US 10155064 B2
- CN 111018716 A
- CN 104262271 B


### Non-patent literature cited in the description


- *Tetrahedron*, 2003, vol. 59 (40), 7983-7996
- *Chemical Communications*, 2010, vol. 46 (1), 142-144
- *RSC Advances*, 2014, vol. 4 (43), 22561-22566
- *Chemical Communications*, 2020, vol. 56 (73), 10662-10665
- *Journal of Organic Chemistry*, 1971, vol. 36, 3042-3044
- *Recueil des Travaux Chimiques des Pays-Bas*, 1938, vol. 57, 265, 276
- *J. Ind. Eng Chem.*, 2000, vol. 39, 4011
- *Canadian Journal of Chemistry*, 1989, vol. 67, 1650-1656
- *Journal of the Chemical Society*, 1938, 1588-1593
- *Recueil des Travaux Chimiques des Pays-Bas*, 1938, vol. 57, 265-276
- *Tur. J. Chem.*, 2001, vol. 25, 293
- *Journal of the American Chemical Society*, 2012, vol. 134 (32), 13145-13147
- *Pure Appl Chem*, 1976, vol. 45, 11-30
- *Research Disclosure Database Number*, 01 August 2014, vol. 2014, 605005